# EUROPEAN PATENT APPLICATION

(11) **EP 2 888 995 A2**
(43) Date of publication of application: **01.07.2015**
(21) Application number: 14200049.6
(22) Date of filing: 23.12.2014
(51) Int. Cl.: A61B 5/00, G06F 21/32, H04L 29/06

(54) **Biometric information measuring apparatus**

(30) Priority: 25.12.2013 JP 2013266626; 25.12.2013 JP 2013266627; 25.12.2013 JP 2013266628; 09.09.2014 JP 2014182993
(71) Applicant: Seiko Epson Corporation, Tokyo 163-0811 (JP)
(72) Inventor: Shimizu, Yoshiaki, Nagano, 392-8502 (JP); Nakajima, Kenichi, Nagano, 392-8502 (JP)
(74) Representative: Hoffmann Eitle

(57) **Abstract**

A measuring apparatus includes a case unit that is provided with a detection unit that detects biometric information; a band unit that fixes the case unit to a living body; and a buckle unit that is connected to the band unit to form a ring shape and has an adjustable length.

## Description

### BACKGROUND

### 1. Technical Field

The present invention relates to a biometric information measuring apparatus.

### 2. Related Art

In the related art, a measuring apparatus that is mounted on the wrist or the like through a band unit or the like and measures biometric information such as the pulse of a person who wears the measuring apparatus, or a watch type electronic apparatus having such a biometric information measuring function is known (see JP-A-2006-312010, JP-A-2008-168054, and JP-A-2008-167893, for example). In such an apparatus (measuring apparatus or electronic apparatus), a case unit (main body unit) provided with a display unit is mounted on the wrist by two band members that extend from upper and lower portions of the case unit. Further, a detection unit (sensor) that optically detects the pulse is disposed on a rear surface of the case unit (opposite surface to the display unit).

Further, in such an apparatus, in order to stably measure the biometric information, it is necessary that the detection unit come into close contact with the arm. Specifically, even if a in portion on which the apparatus is mounted, for example, the size or cross-sectional shape of the wrist is different for each person who wears the apparatus, it is necessary that the apparatus be mountable in a state of being in close contact with the case unit. Further, in order to continuously measure the biometric information, it is necessary that the entire apparatus including the band unit have a small size and a light weight so that the wearer can conveniently wear the apparatus with little burden for a long time. Furthermore, in order to motivate the wearer to wear the apparatus for a long time, it is preferable that the appearance of the entire apparatus including the band unit be pleasant.

Further, JP-A-2008-61842 discloses a structure in which a buckle is attached to a band unit of a watch. Specifically, in the end portions of two band members, a fold-type buckle that connects both of the band members is attached. According to this configuration, prior to wearing, the buckle is in an opened (extended) state, in which a large annular opening is formed by the case unit, the two band members, and the buckle. During wearing, the wearer inserts the hand into the opening with the fingers being closed and folds the buckle in order to wear the apparatus on the arm.

However, in the buckle for the watch according to the related art, it is difficult for the apparatus to secure the close-contact for stable measurement of the biometric information.

First, JP-A-2006-312010 does not disclose a fixing method (structure) for the end portions of the two band members. If a configuration in which plural holes are formed in one band member and a pin-like buckle is attached to the other band member is used, whenever the wearer wears the apparatus, a hole where the buckle is inserted may be different, which makes it difficult to stabilize measurement conditions and reduces the convenience of use.

Further, in JP-A-2006-312010, a case unit in which a display unit or a switch is provided and the size of a portion thereof that covers a width-directional portion of the arm is large is used. Here, if the display unit is configured to have a large size with respect to the arm, it is difficult to secure the close-contact with respect to the arm having a curved surface. Further, it is presumed that the two band members are attached to upper and lower portions of the case unit as separate parts, but in this configuration, looseness may occur due to assembly tolerances of the connection portions at two locations, and consequently, it is difficult to secure the close-contact with respect to the arm.

Further, a sensor node (apparatus main body) disclosed in JP-A-2006-312010 has a space for an antenna on a front surface thereof in addition to the display unit (monitor) and the switch, and thus has a size equal to or larger than that of a watch. Thus, when the large apparatus main body is mounted on the wrist and the wrist is then pressed to such a degree that the biometric information can be detected, the wearer may feel uncomfortable during a period of continuous use. Further, in addition to the functional characteristics of the apparatus, it is preferable that design characteristics of the apparatus be excellent. Otherwise, the user may not want to wear the apparatus for a long time from the very beginning. In addition, since the apparatus main body protrudes from the band unit, the apparatus main body may be caught in a sleeve or other clothes or may contact with an obstacle or the like and be separated or shifted from a measurement portion of the wearer, which is problematic. Accordingly, it is desirable to provide a biometric information measuring apparatus capable of being conveniently mounted for a long time while reducing the burden to the wearer and continuously measuring the biometric information in a stable state.

Further, a band unit for a living body measuring apparatus disclosed in JP-A-2008-168054 has an approximate U shape of which a part is opened. Thus, when a wearer moves suddenly or when the apparatus physically contacts an obstacle or the like, the apparatus may be separated or shifted from a measurement portion of the wearer, which is problematic.

Further, in a biometric information managing apparatus disclosed in JP-A-2008-167893, in addition to a watch-like apparatus main body mounted on the wrist, a configuration in which a separate sensor unit is mounted on a finger is used. In order to increase close-contact of the sensor unit, the sensor unit is mounted while tightening the root of the finger. Thus, if the sensor unit is mounted for a long time, the finger becomes difficult to use, for example, which may cause the wearer to feel uncomfortable.

Furthermore, JP-A-2008-61842 discloses a technique relating to a fixing (mounting) structure of a watch, but since a mounting condition necessary for the watch is simpler than a condition necessary for the measuring apparatus, it is difficult to secure close-contact necessary for stably measuring the biometric information. That is, in the case of the watch, in general, room (gap) for insertion of one finger after wearing is maintained in a band unit. This is because it is sufficient if the watch can be mounted so that a main body of the watch can be prevented from being rotated on the front surface of the arm or from being separated from the arm during use of the watch.

### SUMMARY

An advantage of some aspects of the invention is to solve at least a part of the problems described above, and the invention can be implemented as the following application examples, aspects or forms.

### Application Example 1

A biometric information measuring apparatus according to this application example includes: a case unit that is provided with a detection unit that detects biometric information; a band unit that fixes the case unit to a living body; and a buckle unit that is connected to the band unit.

With this configuration, when wearing the biometric information measuring apparatus, by extending the length of the buckle unit, inserting the hand into a large opening formed in this state, for example, with the hand being closed, and then shortening the length of the buckle unit at a mounting position of the arm, it is possible to mount the biometric information measuring apparatus. Here, the size of the opening is adjusted in advance to a size suitable for a wearer at a connection (attachment) position of the buckle unit to the band unit. The size suitable for the wearer refers to a size relationship for bringing the detection unit (case unit) into close contact with a detection position of the arm during wearing. Further, the close contact refers to accurate fixation of the detection unit to the detection position of the arm with approximately the same pressure (pressing force) even though mounting and detaching of the biometric information measuring apparatus is repeated. In other words, the close contact refers to contact of the detection unit to the detection position of the arm with high reproducibility.

According to the biometric information measuring apparatus with this configuration, it is possible to secure close-contact (fit property) necessary for stably measuring the biometric information.

Accordingly, it is possible to provide a biometric information measuring apparatus capable of stably measuring biometric information even under repetitive mounting and detaching. Further, it is possible to adjust the close-contact by a simple method of shortening the length of the buckle unit after wearing. In addition, even in a state where the length of the buckle unit is extended, since the entire apparatus maintains a ring shape, there is no concern about losing the apparatus unless the apparatus is separated from the arm, and it is possible to prevent the apparatus from being broken due to being dropped.

### Application Example 2

In the biometric information measuring apparatus according to the application example described above, it is preferable that a hole portion is provided in the band unit, the case unit is fitted in the hole portion, the band unit includes a first band portion that extends from the hole portion toward one end thereof and a second band portion that extends from the hole portion toward the other end thereof, in an extension direction of the band unit, and the buckle unit is attached to connect the first band portion and the second band portion.

With this configuration, since the band unit and the case unit are integrally formed, the fit to the arm becomes excellent. Further, it is possible to achieve an integrated design, and to provide a biometric information measuring apparatus having a pleasant appearance.

### Application Example 3

In the biometric information measuring apparatus according to the application example described above, it is preferable that the band unit is formed of a material including resin having elasticity, and the buckle unit is formed of metal.

With this configuration, since the band unit is formed of the resin having elasticity, it is possible to fix the case unit to the arm with a suitable pressing force. Accordingly, it is possible to bring the detection unit into close contact with the detection position. Further, since the buckle unit is formed of metal, it is possible to secure the strength and durability thereof.

### Application Example 4

In the biometric information measuring apparatus according to the application example described above, it is preferable that the buckle unit is a fold-type buckle that includes a hinge portion, and the size of an inner circumference of a ring shape formed by the case unit, the band unit and the buckle unit in an extended state, in an opened state of the buckle unit, is in a range of 200 mm to 330 mm.

With this configuration, most adults can wear and take off the biometric information measuring apparatus with ease.

### Application Example 5

In the biometric information measuring apparatus according to the application example described above, it is preferable that the size of the inner circumference of the ring shape formed by the case unit, the band unit and the buckle unit, in a mounted state where the buckle unit is folded, is in a range of 130 mm to 220 mm.

With this configuration, most adults can wear and take off the biometric information measuring apparatus with ease.

### Application Example 6

In the biometric information measuring apparatus according to the application example described above, it is preferable that the size of the inner circumference of the ring shape formed by the case unit, the band unit and the buckle unit in the mounted state is shorter than the size of the outer circumference of a mounting portion of the living body to which the apparatus is applied.

With this configuration, since a tightening force is generated when the biometric information measuring apparatus is mounted, it is possible to fix the case unit to the arm with a suitable pressing force. Accordingly, it is possible to bring the detection unit into close contact with the detection position.

### Application Example 7

In the biometric information measuring apparatus according to the application example described above, it is preferable that a difference between the sizes of the inner circumference of the ring shape in the opened state and the mounted state is in a range of 70 mm to 80 mm.

With this configuration, it is possible to easily mount and detach the biometric information measuring apparatus, and to secure close-contact of the detection unit during wearing.

### Application Example 8

In the biometric information measuring apparatus according to the application example described above, it is preferable that the buckle unit includes a first plate and a second plate around the hinge portion, the second plate is connected to the second band portion, is provided with a positioning hole, and is folded to overlap with the second band portion in the mounted state, and a convex portion is provided in the second band portion at a position for being fit in the positioning hole.

With this configuration, since the convex portion of the second band portion is fitted in the positioning hole of the second plate when the biometric information measuring apparatus is mounted, it is possible to suppress positional shift of the band unit in the width direction.

### Application Example 9

In the biometric information measuring apparatus according to the application example described above, it is preferable that the first plate is connected to the first band portion, and includes a hook portion for engagement with the second plate in the folded state, and a concave portion is provided in the second band portion at a position that overlaps the hook portion in the mounted state.

With this configuration, since the hook portion of the first plate and the concave portion of the second band portion overlap each other, it is possible to remove a projection due to the hook portion when the biometric information measuring apparatus is mounted. Accordingly, it is possible to suppress floating of the second band portion.

### Application Example 10

In the biometric information measuring apparatus according to the application example described above, it is preferable that a plurality of adjustment holes for position adjustment is provided in the first band portion along the extension direction, a hook portion for engagement with the adjustment holes is provided in the first plate, and the plurality of adjustment holes is provided to form at least one row along the extension direction, and the number of the hook portion is the same of the number of the row.

With this configuration, since the engagement configuration is preferably formed by two rows, it is possible to securely connect the first band portion and the first plate, compared with a case where the engagement configuration is formed by one row.

### Application Example 11

In the biometric information measuring apparatus according to the application example described above, it is preferable that a first connecting portion for connection to the second band portion is provided in the second plate, a plurality of second connecting portions is provided in the second band portion along the extension direction, and the second plate is fixed to the second band portion through any one of the plurality of second connecting portions.

With this configuration, on the side of the second plate, it is similarly possible to perform the length adjustment in the initial setting.

### Application Example 12

In the biometric information measuring apparatus according to the application example described above, it is preferable that the detection unit includes a first light emitting portion, a second light emitting portion, and a light receiving portion.

With this configuration, light emitted from the light emitting portions can be reflected from the skin or the like without being absorbed in the skin, and can directly reach the light receiving portion. That is, most of the light emitted from the light emitting portions can be directed toward the skin, and the reflected light can be directly incident to the light receiving portion without intervention of an air layer or the like.

### Application Example 13

In the biometric information measuring apparatus according to the application example described above, it is preferable that the detection unit includes a carrier portion, and the first light emitting portion, the second light emitting portion, and the light receiving portion are disposed on a surface of the carrier portion.

With this configuration, most of the light emitted from the light emitting portions can be directed toward the skin, and the reflected light can be directly incident to the light receiving portion without intervention of an air layer or the like. In other words, since the light receiving portion is in close contact with the skin, it is possible to provide a structure in which a gap is not easily generated between a top surface (light receiving surface) of the light receiving portion and the skin. Thus, it is possible to suppress light that is a noise source, such as external light, from being incident to the top surface.

### Application Example 14

In the biometric information measuring apparatus according to the application example described above, it is preferable that the distance between the carrier portion and a top surface of the light receiving portion is larger than the distance between the carrier portion and a top surface of the first light emitting portion.

With this configuration, light from the light emitting portions that does not pass through the skin, for example, light that is directly incident to the light receiving portion from the light emitting portions cannot reach the top surface of the light receiving portion.

### Aspect 1

A biometric information measuring apparatus according to this aspect of the invention includes: a case unit that is provided with a detection unit that detects biometric information to a living body; and a band unit that fixes the case unit to the living body. In a side view of the case unit, an outer surface of the case unit has a first curved surface portion, the band unit has an outer surface having a second curved surface portion on opposite sides of the case unit, and the radius of curvature of the second curved surface portion is smaller than the radius of curvature of the first curved surface portion.

With this configuration, in the side view, the outer surface of the case unit has the first curved surface portion, the band unit has the outer surface having the second curved surface portion having the radius of curvature smaller than that of the first curved surface portion on the opposite sides of the case unit. Accordingly, the band unit is curved toward a living body with reference to a curved surface obtained by virtually extending the first curved surface portion, at portions thereof positioned on the opposite sides of the case unit. Thus, a biasing force that presses the case unit from the opposite sides and a biasing force that causes portions of the band unit that extend on the opposite sides of the case unit to be directed toward the living body are applied to the band unit. Thus, in the mounted state, the biometric information measuring apparatus can be easily fitted to a corresponding measurement position (for example, wrist or the like) of a wearer. Further, it is possible to suppress the burden (discomfort) of the wearer even during a period of continuous use, and to measure the biometric information in a stable state.

### Aspect 2

In the biometric information measuring apparatus according to the aspect described above, it is preferable that opposite end portions of the case unit are projected toward an inner surface of the case unit, and the band unit has convex portions that are projected toward the inner surface of the case unit in the vicinity of the end portions of the case unit.

With this configuration, the band unit has the convex portions that are projected toward the inner surface of the case unit in portions thereof adjacent to portions that are projected toward the inner surface of the case unit at the opposite ends of the case unit. Thus, in a state where the opposite end portions of the case unit are interposed between the convex portions of the band unit, the window portion can be in contact with the living body. Accordingly, compared with a case where the band unit is not provided with the convex portions, it is possible to maintain the case unit in close contact with the measurement portion of the living body in a more stable state, and thus, even during a period of continuous use of the biometric information measuring apparatus, it is possible to measure the biometric information in a stable state.

### Aspect 3

In the biometric information measuring apparatus according to the aspect described above, it is preferable that an opening portion is formed in the band unit, and the case unit is fitted in the opening portion.

With this configuration, since the case unit is fitted in the opening portion provided in the band unit, the case unit can be fixed to the band unit, and the periphery of the case unit can be protected by the band unit. Further, a sense of unity in the appearance of the case unit and the band unit can be enhanced.

### Aspect 4

In the biometric information measuring apparatus according to the aspect described above, it is preferable that the case unit is detachably fitted in the opening portion.

With this configuration, since the band unit is detachably fitted in the case unit, when the band unit is damaged, for example, the band unit can be exchanged. Further, when plural band units having different lengths are prepared, the band units can be exchanged according to the size of the measurement portion of the wearer. In addition, it is possible to diversify an external design by exchanging band units having different colors, shapes or the like.

### Aspect 5

In the biometric information measuring apparatus according to the aspect described above, it is preferable that the case unit includes a window portion for detecting the biometric information by the detection unit on the side of the inner surface, and the window portion is exposed in the opening portion.

With this configuration, the window portion for detecting the biometric information disposed on the side of the inner surface of the case unit is exposed. Thus, since the window portion is directed toward the living body without being blocked in the mounted state, it is possible to detect the biometric information in a stable state.

### Aspect 6

In the biometric information measuring apparatus according to the aspect described above, it is preferable that a corner portion on the side of the inner surface in the band unit is formed to have a curved surface, in a cross-sectional view of the band unit.

With this configuration, in the cross-sectional view of the band unit, the corner portion of the band unit on the side of the measurement portion of the wearer is formed to have the curved surface. Thus, even though the measurement portion of the wearer is tightened to such a degree that the biometric information can be detected by the band unit, since the corner portion contacting the measurement portion is formed to have the curved surface, it is possible to suppress the burden (discomfort) of the wearer during a period of continuous use.

### Aspect 7

In the biometric information measuring apparatus according to the aspect described above, it is preferable that a groove is formed on the side of the inner surface of the band unit.

With this configuration, the groove is provided on the side of the inner surface of the band unit that includes the measurement portion. Thus, it is possible to reduce a substantial contact area of the band unit in contact with the measurement portion of the wearer in the mounted state. Thus, it is possible to improve air circulation to the measurement portion and to release sweat from the measurement portion, to thereby improve fit of the wearer. Further, by providing the groove, it is possible to prevent deviation of the biometric information measuring apparatus in the mounted state.

### Aspect 8

In the biometric information measuring apparatus according to the aspect described above, it is preferable that the band unit includes a first band portion that extends from the case unit in a first direction and is provided with plural adjustment hole portions arranged in a row, and a second band portion that extends from the case unit in a second direction opposite to the first direction, and a buckle unit that connects the first band portion to the second band portion, the buckle unit includes a first buckle portion that is connected to the first band portion in any one adjustment hole portion selected from the plural adjustment hole portions, and a second buckle portion that is supported to be rotatable with respect to the first buckle portion by a hinge portion and is connected to the second band portion, and the buckle unit enters a mounted state where the apparatus is mounted on the living body as the first buckle portion and the second buckle portion are folded so that the second buckle portion overlaps the first buckle portion on a side opposite to the living body, and enters an opened state where the apparatus can be detached from the living body by expanding the folded first buckle portion and second buckle portion.

With this configuration, in the opened state where the first buckle portion and the second buckle portion of the buckle unit are expanded, the biometric information measuring apparatus can be mounted on or detached from the living body in a state where the first band portion is connected to the second band portion. Thus, it is possible to prevent mistaken dropping of the biometric information measuring apparatus during mounting and detaching. Further, by selecting any one of the plural adjustment hole portions, the length of the band unit is adjusted according to the measurement portion (for example, length, shape or the like of the circumference of the wrist) of the living body, to thereby appropriately adjust the tightening force with respect to the measurement portion. Further, it is possible to mount or detach the biometric information measuring apparatus on or from the living body without shifting the selected adjustment hole portion. Thus, it is possible to maintain the adjusted tightening force even though the mounting and detaching of the biometric information measuring apparatus to and from the living body is repeated.

### Aspect 9

In the biometric information measuring apparatus according to the aspect described above, it is preferable that in the mounted state, the first band portion is disposed to overlap the first buckle portion on the side of the first buckle portion toward the living body, and the position of the adjustment hole portion where the first band portion is connected to the first buckle portion can be set between a first position where a length from the adjustment hole portion to a tip portion of the first band portion is the maximum and a second position where the length from the adjustment hole portion to the tip portion is the minimum, and even when the first band portion is connected to the first buckle portion at the first position, and the tip portion is disposed close to the first buckle portion with reference to the case unit on the side of the convex portion of the second band portion.

With this configuration, in a state where the band unit is connected in a ring shape with the buckle unit, if the first band portion is connected to the first buckle portion in the first position, the length of the first band portion from the position of the adjustment hole portion to be connected to the first band portion to the tip portion becomes the maximum. That is, if the first band portion is connected to the first buckle portion in the first position, the diameter of the band unit connected in the ring shape becomes the smallest, and the tip portion of the first band portion disposed on the side of the living body (inner surface) with reference to the buckle unit and the second band portion becomes the closest to the case unit on the side of the convex portion of the second band portion. Even though the first band portion is connected to the first buckle portion in the first position, since the tip portion of the first band portion is disposed close to the first buckle portion with reference to the case unit where the window portion for detecting the biometric information is provided, the first band portion does not intervene between the case unit and the living body, and thus does not block the window portion. Accordingly, even in the mounted state where the diameter of the band unit connected in the ring shape becomes the smallest, it is possible to measure the biometric information in a stable state.

### Aspect 10

In the biometric information measuring apparatus according to the aspect described above, it is preferable that even when the first band portion is connected to the first buckle portion in the first position, the tip portion is disposed close to the first buckle portion with reference to the convex portion of the second band portion.

According to this configuration, even though the first band portion is connected to the first buckle portion in the first position, since the tip portion of the first band portion is disposed close to the first buckle portion with reference to the convex portion of the second band portion, the first band portion does not intervene between the convex portion of the second band portion and the living body. Accordingly, even in the mounted state where the diameter of the band unit connected in the ring shape becomes the shortest, it is possible to measure the biometric information in a stable state.

### Aspect 11

In the biometric information measuring apparatus according to the aspect described above, it is preferable that even when the first band portion is connected to the first buckle portion in the second position, the tip portion is disposed close to the convex portion of the second band portion with reference to the first buckle portion.

With this configuration, in a state where the band unit is connected in the ring shape by the buckle unit, if the first band portion is connected to the first buckle portion in the second position, the length of the first band portion from the position to be connected to the first buckle portion to the tip portion becomes the minimum. That is, the tip portion of the first band portion disposed on the side of the living body with reference to the buckle unit becomes closest to the first buckle portion. Even when the first band portion is connected to the first buckle portion in the second position, since the tip portion of the first band portion is disposed close to the convex portion of the second band portion with reference to the first buckle portion, the first band portion intervenes between the first buckle portion and the living body. Accordingly, even in the mounted state where the diameter of the band unit connected in the ring shape becomes the largest, since the first buckle portion is not in contact with the measurement portion of the living body, it is possible to suppress the burden (discomfort) of the wearer from occurring.

### Aspect 12

In the biometric information measuring apparatus according to the aspect described above, it is preferable that the tip portion is curved toward a side close to the living body in the opened state.

With this configuration, the tip portion of the first band portion disposed on the side (inner side) of the first buckle portion toward the living body is bent toward a side separated from the side of the living body (inner side), that is, from the first buckle portion in the opened state. Thus, when the first buckle portion and the second buckle portion are folded from the opened state to be mounted on the measurement portion of the living body, it is possible to prevent the tip portion of the first band portion from bending toward the first buckle portion (outer side) to be wound between the first band portion and the first buckle portion or between the second buckle portion and the second band portion.

### Aspect 13

In the biometric information measuring apparatus according to the aspect described above, it is preferable that the tip portion of the second band portion is bent toward a side close to the living body in the mounted state.

With this configuration, the tip portion of the second band portion disposed on the side (outer side) of the second buckle portion opposite to the living body is bent toward the living body (inner side), that is, toward a side close to the second buckle portion in the mounted state. Thus, it is possible to prevent the tip portion of the second band portion from being caught in a sleeve or other clothes in the mounted state.

### Aspect 14

A biometric information measuring apparatus according to this aspect of the invention includes: a case unit that includes a detection unit that detects biometric information; and a band unit that includes a main body portion that accommodates the case unit, a first band portion that extends from the main body portion in a first direction and a second band portion that extends from the main body portion in a second direction opposite to the first direction. The band unit includes a third curved surface portion having a first radius of curvature that forms an outer surface of the main body portion, and a second curved surface portion that extends in the first direction and the second direction, has a radius of curvature smaller than the first radius of curvature, and is provided to be continuous with the third curved surface portion, in a side view.

With this configuration, in the side view, the band unit includes the third curved surface portion that forms the outer surface of the main body portion, and the second curved surface portion that extends in the first and second directions and is provided to be continuous with the third curved surface portion, in which the radius of curvature of the second curved surface is smaller than the first radius of curvature of the third curved surface portion. Accordingly, the band unit is bent toward the living body with reference to a curved surface obtained by virtually extending the third curved surface portion, of portions disposed on opposite sides of the main body portion that accommodate the case unit. Thus, a biasing force that presses the case unit from the opposite sides and a biasing force that causes the portions of the band unit that extend on both sides of the case unit to be directed toward the living body are applied to the band unit. Thus, in the mounted state, since the biometric information measuring apparatus is easily fitted to the measurement portion (for example, wrist or the like) of the wearer, it is possible to reduce the burden (discomfort) of the wearer even during a period of continuous use, and to measure the biometric information in a stable state.

### Aspect 15

In the biometric information measuring apparatus according to the aspect described above, it is preferable that the case unit includes a window portion for detecting the biometric information with the detection unit at a center portion thereof in the side view, and a bottom surface that extends in the first and second directions, and the bottom surface includes a fourth curved surface portion having a radius of curvature larger than the first radius of curvature between the center portion and opposite end portions thereof.

With this configuration, the fourth curved surface portion is provided between the center portion on the bottom surface of the case unit and the opposite end portions, and the radius of curvature of the fourth curved surface portion is larger than the first radius of curvature of the third curved surface portion that forms the outer surface of the main body portion of the band unit. Since the radius of curvature of the fourth curved surface portion on the side of the inner surface that contacts with the living body in the biometric information measuring apparatus is larger than the radius of curvature of the third curved surface portion of the main body portion, the thickness of the detection unit can be absorbed by expanding the side of the third curved surface portion (outer surface), and thus, reduction of the close-contact to the living body of the biometric information measuring apparatus is prevented.

### Form 1

A biometric information measuring apparatus according to this form of the invention includes: a case unit that is provided with a detection unit that detects biometric information of a living body; and a band unit that fixes the case unit to the living body. In a side view of the case unit, at least a part of a side portion of the case unit is covered by the band unit.

With this configuration, in the side view of the case unit, at least a part of the side portion of the case unit is covered by the band unit. Accordingly, the width of the band unit in the side portion of the case unit is larger than the width of the case unit. Thus, since the side portion of the case unit does not protrude from the band unit, the case unit is suppressed from being caught in a sleeve or other clothes or contacting with an obstacle or the like. Further, even though the portion covered by the band unit contacts an obstacle or the like, since shock due to the contact is moderated by the band unit, it is possible to suppress the case unit from being separated or deviated from a measurement portion of a wearer. Thus, even during a period of continuous use of the biometric information measuring apparatus, it is possible to reduce the burden of the wearer (living body), and to measure the biometric information in a stable state. Further, by covering the side portion of the case unit with the band unit, it is possible to provide a sense of unity in the appearance of the case unit and the band unit, and to diversify an external design by combination of the case unit and the band unit, thereby making the appearance of the biometric information measuring apparatus nice.

### Form 2

In the biometric information measuring apparatus according to the form described above, it is preferable that an outer circumference of an outer surface of the case unit is covered by the band unit.

With this configuration, the outer circumference of the outer surface of the case unit is covered by the band unit. Thus, the outer circumference of the outer surface of the case unit is suppressed from being caught in a sleeve of clothes or contacting with an obstacle or the like. Further, since shock due to the contact with the obstacle or the like is moderated, it is possible to suppress the case unit from being separated or deviated from the measurement portion of the wearer more efficiently. Further, by covering the outer circumference of the case unit by the band unit, it is possible to make the appearance of the biometric information measuring apparatus fine.

### Form 3

In the biometric information measuring apparatus according to the form described above, it is preferable that opposite end portions of the case unit in an extension direction of the band unit is projected toward the inner surface, and the band unit includes convex portions that are projected toward the inner surface of the case unit in the vicinity of the end portions of the case unit.

With this configuration, the band unit includes the convex portions that are projected toward the inner surface of the case unit in the portions thereof adjacent to portions that are projected toward the inner surface of the case unit at the opposite ends of the case unit. Thus, in a state where the opposite end portions of the case unit are interposed between the convex portions of the band unit, the case unit can be in contact with the living body. Accordingly, compared with a case where the band unit is not provided with the convex portions, it is possible to maintain the case unit in close contact with the measurement portion of the wearer in a more stable state, and thus, even during a period of continuous use of the biometric information measuring apparatus, it is possible to measure the biometric information in a stable state.

### Form 4

In the biometric information measuring apparatus according to the form described above, it is preferable that an opening portion is formed in the band unit, and the case unit is fitted in the opening portion.

With this configuration, since the case unit is fitted in the opening portion provided in a middle portion of the band unit in the extension direction, the case unit can be fixed to the band unit, and the periphery of the case unit can be protected by the band unit. Further, a sense of unity in the appearance of the case unit and the band unit can be enhanced.

### Form 5

In the biometric information measuring apparatus according to the form described above, it is preferable that the case unit includes a window portion for detecting the biometric information by the detection unit on the side of the inner surface, and the window portion is exposed in the opening portion.

With this configuration, the window portion for detecting the biometric information disposed on the side of the inner surface of the case unit is exposed. Thus, since the window portion is directed to the measurement portion of the wearer without being blocked in the mounted state, it is possible to detect the biometric information in a stable state.

### Form 6

In the biometric information measuring apparatus according to the form described above, it is preferable that the window portion protrudes toward the inner surface with reference to the end portions.

With this configuration, since the window portion protrudes toward the inner surface with reference to the opposite end portions of the case unit, the window portion is reliably pressed against the measurement portion of the wearer in the mounted state, and thus, it is possible to measure the biometric information in a stable state.

### Form 7

In the biometric information measuring apparatus according to the form described above, it is preferable that the end portions protrude toward the inner surface with reference to the window portion.

With this configuration, since the window portion does not protrude toward the inner surface with reference to the opposite end portions of the case unit, the window portion is not excessively pressed against the measurement portion of the wearer in the mounted state, and thus, it is possible to enhance a fitting characteristic to the measurement portion of the wearer.

### Form 8

In the biometric information measuring apparatus according to the form described above, it is preferable that the band unit includes a first band portion that extends from the case unit in one direction thereof and is provided with plural adjustment hole portions, and a second band portion that extends from the case unit in the other direction, and further includes a buckle unit that connects the first band portion to the second band portion, the buckle unit includes a first buckle portion that is connected to the first band portion in any one adjustment hole portion selected from the plural adjustment hole portions, and a second buckle portion that is supported to be rotatable with respect to the first buckle portion by a hinge portion and is connected to the second band portion, and the buckle unit enters a mounted state where the apparatus is mounted on the living body as the first buckle portion and the second buckle portion are folded so that the second buckle portion overlaps the first buckle portion on a side opposite to the living body, and enters an opened state where the apparatus can be detached from the living body by expanding the folded first buckle portion and second buckle portion.

With this configuration, in the opened state where the first buckle portion and the second buckle portion of the buckle unit are expanded, the biometric information measuring apparatus can be mounted on or detached from the measurement portion of the wearer in a state where the first band portion is connected to the second band portion. Thus, it is possible to prevent mistaken dropping of the biometric information measuring apparatus in mounting and detaching. Further, by selecting any one of the plural adjustment hole portions, the length of the band unit is adjusted according to the measurement portion of the wearer (for example, length, shape or the like of the circumference of the wrist), to thereby appropriately adjust the tightening force with respect to the measurement portion. Further, it is possible to mount or detach the biometric information measuring apparatus on or from the measurement portion of the wearer without shift of the selected adjustment hole portion. Thus, it is possible to maintain the adjusted tightening force even though the mounting and detaching of the biometric information measuring apparatus to and from the measurement portion of the wearer is repeated.

### Form 9

In the biometric information measuring apparatus according to the form described above, it is preferable that an arrangement pitch of the plural adjustment hole portions is not uniform.

With this configuration, since the length from the adjustment hole portion in the first band portion to the case unit is shortened as the adjustment hole portion goes closer to the case unit when the first band portion is connected to the first buckle unit, a tensile stress of the first band portion increases with respect to the same distortion amount (extending length). Thus, if the arrangement pitch of the plural adjustment hole portions is uniform, as the adjustment hole portion for connection goes closer to the case unit, the increase amount of the tightening force with respect to the measurement portion generated when the adjustment hole portion shifts one by one becomes larger. According to this form, since the arrangement pitch of the plural adjustment hole portions is not uniform, for example, if a configuration in which the arrangement pitch of the adjustment hole portions becomes smaller as the adjustment hole portion goes close to the case unit is used, it is possible to suppress the increase amount of the tightening force with respect to the measurement portion generated when the adjustment hole portion shifts one by one. Thus, it is possible to suppress variation of the tightening forces to the measurement portion even though the measurement portions (length, shape or the like of the circumference of the wrist) are different according to the wearers, and to mount the biometric information measuring apparatus. As a result, with respect to various wearers, it is possible to suppress the case unit from being separated or deviated from the measurement portion of the wearer, and to reduce the burden of the wearer during a period of continuous use.

### Form 10

In the biometric information measuring apparatus according to the form described above, it is preferable that signs are respectively given to the plural adjustment hole portions, in the first band portion.

With this configuration, the signs are respectively given to the plural adjustment hole portions, in the first band portion. Thus, it is possible to individually specify the adjustment hole portions with reference to the signs, after the optimal adjustment hole portion is selected to connect the first band portion to the first buckle portion, and thus, even though the connection is released, it is possible to easily perform re-connection using the previously selected optimal adjustment hole portion.

### Form 11

In the biometric information measuring apparatus according to the form described above, it is preferable that in the mounted state, the first band portion is disposed to overlap the first buckle portion on the side of the first buckle portion toward the living body, the second band portion is disposed to overlap the second buckle portion on a side of the second buckle portion opposite to the living body, and the width in the portion of the second band portion that overlaps the second buckle portion is larger than the widths of the first buckle portion and the second buckle portion.

With this configuration, in the mounted state, the first band portion, the first buckle portion, the second buckle portion, and the second band portion are sequentially disposed from the side (inner side) of the living body to the side (outer side) opposite to the living body to overlap each other. Further, the width of the second band portion disposed on the outermost side is larger than the widths of the first buckle portion and the second buckle portion. Thus, since the first buckle portion and the second buckle portion are covered by the second band portion when seen from the outside, it is possible to prevent the first buckle portion and the second buckle portion from being caught in a sleeve or the like of clothes or from contacting with an obstacle or the like, to thereby make the appearance fine.

### Form 12

In the biometric information measuring apparatus according to the form described above, it is preferable that the tip portion of the first band portion is bent toward a side close to the living body in the opened state.

With this configuration, the tip portion of the first band portion disposed on the side (inner side) of the first buckle unit toward the living body is bent toward the living body (inner side), that is, toward a side separated from the first buckle portion in the opened state. Thus, when the first buckle portion and the second buckle portion are folded to be mounted on the measurement portion of the living body from the opened state, it is possible to prevent the tip portion of the first band portion from bending toward the first buckle portion (outer side) to be wound into between the first band portion and the first buckle portion or into between the second buckle portion and the second band portion.

### Form 13

In the biometric information measuring apparatus according to the form described above, it is preferable that the tip portion of the second band portion is bent toward a side close to the living body in the mounted state.

With this configuration, the tip portion of the second band portion disposed on the outer side with reference to the second buckle portion with respect to the living body is bent toward the side close to the living body, that is, toward the inner side. Thus, when the first buckle portion and the second buckle portion are folded to be mounted on the living body or in the mounted state, it is possible to prevent the tip portion of the second band portion from being caught in a sleeve or the like of clothes.

### Form 14

In the biometric information measuring apparatus according to the form described above, it is preferable that unevenness machining is performed on a front surface of at least a portion where the first band portion and the second band portion are in contact with each other.

With this configuration, since the unevenness machining (surface texturing) is performed on the front surface of the portion where the first band portion and the second band portion are in contact with each other, a frictional force generated when the first band portion and the second band portion rub against each other is reduced to be easily slipped. Thus, compared with a case where the surface texturing is not performed, it is possible to easily perform the mounting and detaching of the measuring apparatus while suppressing winding of the tip portion of the first band portion and the tip portion of the second band portion when the apparatus is mounted.

### Form 15

In the biometric information measuring apparatus according to the form described above, it is preferable that unevenness machining is performed on a front surface of a portion where the first band portion and the second band portion are in contact with the living body.

With this configuration, the unevenness machining (surface texturing) is performed on the front surface of the portion where the first band portion and the second band portion are in contact with the living body. Thus, compared with a case where the surface texturing is not performed, it is possible to reduce a substantial contact area per unit area of the first band portion and the second band portion to the living body. Thus, it is possible to suppress discomfort of the wearer as the first band portion and the second band portion are in close contact with the living body in the mounted state.

### BRIEF DESCRIPTION OF THE DRAWINGS

The invention will be described with reference to the accompanying drawings, wherein like numbers reference like elements.
Figs. 1A and 1B are diagrams illustrating a schematic configuration of a biometric information measuring apparatus according to Embodiment 1 of the invention.
Fig. 2 is an exploded perspective view illustrating a schematic structure of the biometric information measuring apparatus according to Embodiment 1.
Figs. 3A to 3C are diagrams illustrating a schematic configuration of a case unit and a band unit according to Embodiment 1.
Figs. 4A to 4C are diagrams illustrating a schematic configuration of the case unit and the band unit.
Figs. 5A to 5C are diagrams illustrating a schematic configuration of a buckle unit.
Fig. 6 is a side view in an expanded state.
Fig. 7 is a side view in a mounted state.
Fig. 8A is a table of measured data, and Fig. 8B is a diagram illustrating a state where the hand is closed.
Fig. 9 is a diagram illustrating a schematic configuration of a band unit according to Modification example 1.
Fig. 10 is an exploded perspective view illustrating a schematic configuration of a biometric information measuring apparatus according to Modification example 2.
Figs. 11A to 11C are diagrams illustrating a schematic configuration of a case unit and a band unit according to Embodiment 2.
Fig. 12 is a diagram illustrating a schematic configuration of the case unit and the band unit.
Figs. 13A and 13B are diagrams illustrating a schematic configuration of the band unit and a buckle unit.
Figs. 14A to 14C are diagrams illustrating the shape of the band unit.
Fig. 15 is a view illustrating comparison of band units having different lengths.
Fig. 16 is a diagram illustrating a schematic configuration of a case unit and a band unit according to Modification example 6.
Fig. 17 is a diagram illustrating a schematic configuration of a band unit according to Modification example 7.
Fig. 18 is a diagram illustrating a schematic configuration of a band unit according to Modification example 8.
Fig. 19 is a cross-sectional view illustrating a related art example of a biometric information measuring apparatus according to Embodiment 3.
Fig. 20 is a perspective view illustrating the biometric information measuring apparatus according to Embodiment 3.
Fig. 21 is a front view illustrating a biometric information measuring apparatus according to Embodiment 4.
Fig. 22 is a perspective view illustrating a biometric information measuring apparatus according to Embodiment 5.
Fig. 23 is a cross-sectional view illustrating a biometric information measuring apparatus according to Embodiment 6.
Fig. 24 is a flowchart illustrating a method for manufacturing the biometric information measuring apparatus according to Embodiment 3 to Embodiment 6.

### DESCRIPTION OF EXEMPLARY EMBODIMENTS

Hereinafter, embodiments of the invention will be described with reference to the accompanying drawings. In the following drawings, since respective layers or portions have sizes capable of being recognized on the drawings, scales of the respective layers or portions are different from actual sizes.

### Embodiment 1

### Outline of biometric information measuring apparatus

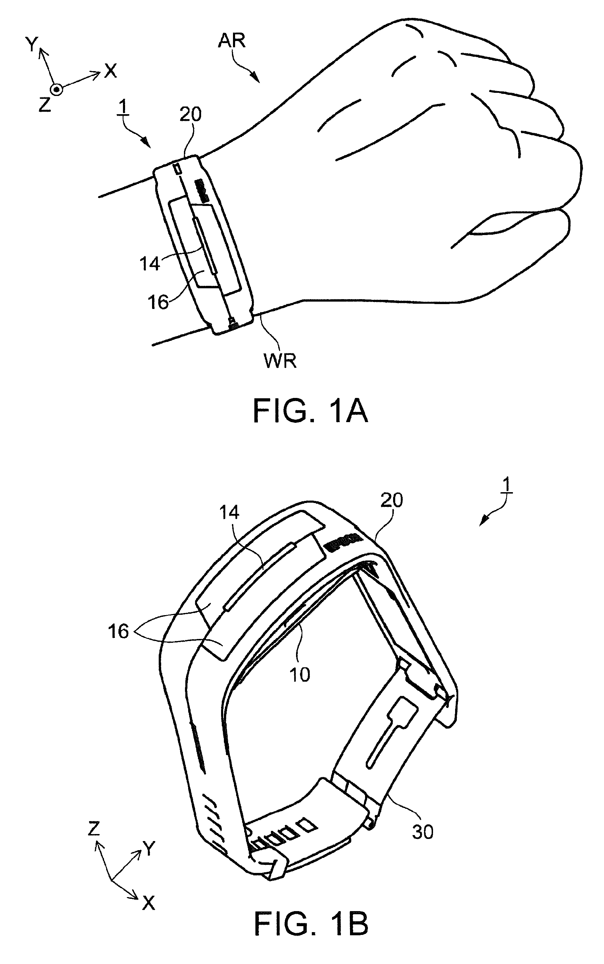

Fig. 1A is a diagram illustrating a mounted state indicating that a biometric information measuring apparatus is mounted on a living body according to Embodiment 1. Fig. 1B is a diagram illustrating an opened state where the biometric information measuring apparatus is detached from the living body.

A biometric information measuring apparatus (hereinafter, referred to as a measuring apparatus) 1 according to Embodiment 1 is an electronic apparatus that is mounted on a living body (for example, a human body) of which biometric information is to be measured and measures the biometric information such as the pulse. As shown in Fig. 1A, the measuring apparatus 1 is mounted on a measurement portion (wrist and the like) of a person (living body) who wears the apparatus like a watch. Fig. 1A shows a state where the measuring apparatus 1 is mounted on a wrist WR of a left arm AR of the wearer.

In this description, it is assumed that a normal direction of a front surface of the measuring apparatus 1 is a Z-axis direction in which a front side in Fig. 1A is positive. The front surface of the measuring apparatus 1 represents a surface where a light emitting portion 14 is disposed. Further, it is assumed that a length direction of the arm AR which is a direction crossing the Z-axis direction is an X-axis direction in which a tip portion where there are fingers is positive. Further, it is assumed that a width direction of the arm AR which is a direction crossing the Z-axis direction and the X-axis direction is a Y-axis direction in which a little finger side is positive.

In this description, a "front view" represents a view of the measuring apparatus 1 seen from the normal direction (Z-axis direction) of the front surface. Further, a "side view" represents a view of the measuring apparatus 1 seen from the X-axis direction. In addition, an "inner side" or an "inner surface" represents a living body side, that is, a side facing the wrist WR in a state where the measuring apparatus 1 is mounted on the wrist WR, and an "outer side" or an "outer surface" represents a side opposite to the living body, that is, a side opposite to the side facing the wrist WR.

The measuring apparatus 1 includes the light emitting portion 14, instead of a monitor unit (display) for displaying characters, graphics or the like as in a general biometric information measuring apparatus (hereinafter, referred to as a general measuring apparatus) similar to a watch. Further, the measuring apparatus 1 does not include a button or a switch for operation as in the general measuring apparatus. The measuring apparatus 1 measures biometric information in a state where a bottom surface (detection unit) opposite to the front surface of the measuring apparatus 1 is in close contact with the wrist WR. A configuration in which a vibration motor, an alarm or the like is provided instead of the light emitting portion 14 may be used.

As shown in Fig. 1B, the measuring apparatus 1 includes a case unit 10 which is an apparatus main body, a band unit 20 that fixes the case unit 10 to the wrist WR, and a buckle unit 30 connected to the band unit 20.

The band unit 20 covers the front surface side of the case unit 10 along the Y-axis direction, is extended from the inner side of the case unit 10, and is connected by the buckle unit 30.

The buckle unit 30 is formed by a hinge unit in which two metallic plates are connected to each other by a rotating shaft, and has a structure in which the length becomes short when two plates are folded to overlap each other and becomes long when two plates are extended.

That is, the measuring apparatus 1 includes the case unit 10 that is provided with a detection unit that detects the biometric information, the band unit 20 that fixes the case unit 10 to the living body, and the buckle unit 30 that is connected to the band unit 20 to form a ring shape and can adjust the length.

In this way, as both ends of the band unit 20 are connected to each other by the buckle unit 30, the measuring apparatus 1 forms the ring shape in both states of a state of being mounted to the wrist WR shown in Fig. 1A (hereinafter, referred to as a mounted state) and a state of being detached from the wrist WR shown in Fig. 1B (hereinafter, referred to as an opened state).

With such a configuration, when wearing the measuring apparatus 1, the measuring apparatus 1 can be mounted as shown in Fig. 1A by inserting the hand into a large opening portion of the ring shape shown in Fig. 1B with the hand being closed in a state where the buckle unit 30 is extended, and then by folding the buckle unit 30 at the mounting position of the arm so that the length becomes short. Particularly, by applying various designs such as optimization of the configuration and material of the band unit 20 or the size of the ring-shaped opening portion, a configuration in which the detection unit can be accurately fixed to the detection position of the arm with approximately the same pressure (pressing force) even though the mounting and detaching of the measuring apparatus 1 is repeated is realized. Details thereof will be described later.

### Overall configuration of biometric information measuring apparatus

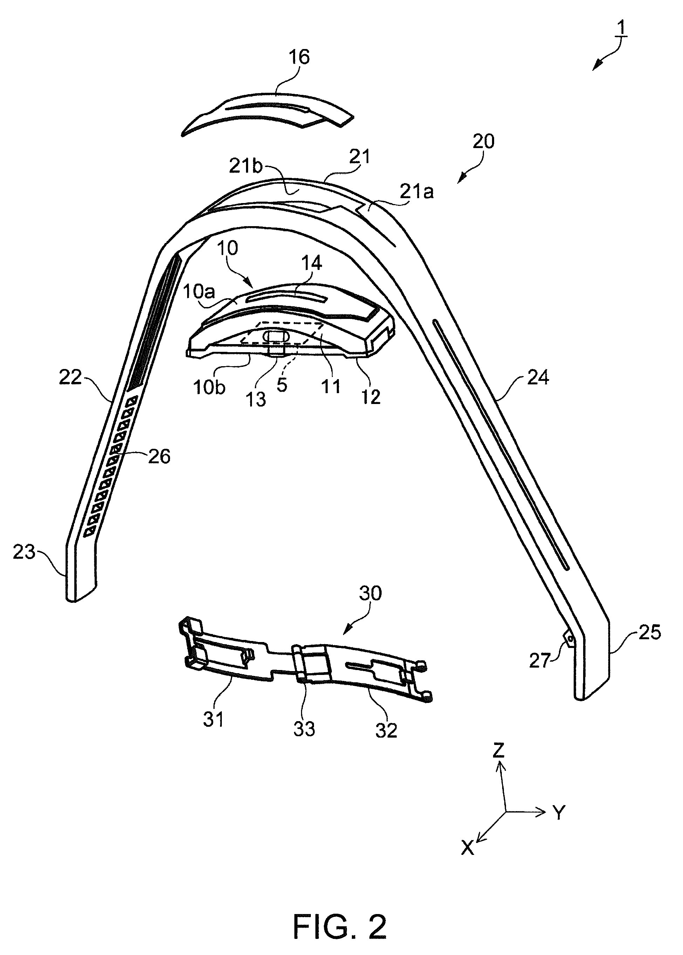

Fig. 2 is an exploded perspective view illustrating a schematic structure of the biometric information measuring apparatus.

As shown in Fig. 2, an opening portion 21b (hole portion) is formed in the band unit 20 in a middle portion thereof in the extension direction. The case unit 10 of a substantially rectangular shape in a plane view is fitted in the opening portion 21b. The case unit 10 is fitted (inserted) into the opening portion 21b from the rear surface side of the band unit 20 in a state where the light emitting portion 14 is directed upward (front surface side). The portion of the band unit 20 where the opening portion 21b is formed in the extension direction is formed to be wider than the both end portions thereof.

The band unit 20 includes a first band portion 22 that extends from the opening portion 21b toward one end thereof, and a second band portion 24 that extends toward the other end thereof. The band unit 20 in a single product (initial) state is formed in an inverted-U shape in which the first band portion 22 and the second band portion 24 drop right and left with the middle portion (opening portion 21b) being a top portion, from the side view thereof.

The case unit 10 includes side portions 11 that extend along the Y-axis direction, and end portions 12 that extend along the X-axis direction, and is formed in a substantially rectangular shape in which the side portions 11 are long sides and the end portions 12 are short sides from the front view. Further, the case unit 10 includes a top surface 10a formed by a convex curved surface on the front surface side of the measuring apparatus 1, and a bottom surface 10b on a side opposite to the top surface 10a, that is, a side facing the wrist WR. A window portion 13 as the detection unit is disposed on the bottom surface 10b. In reality, a sensor that detects the biometric information is a photoelectric pulse wave sensor unit 5 disposed in a recess of the window portion 13, but since a portion that most protrudes toward the wrist WR and has necessary close-contact is the window portion 13, the window portion 13 represents the detection unit. The case unit 10 is formed of a resin material such as polycarbonate (PC), polystyrene (PS) or ABS resin, for example.

The light emitting portion 14 is disposed on the top surface 10a of the case unit 10. The light emitting portion 14 is configured by plural light emitting elements formed of light emitting diodes (LED) or the like, for example. As a preferred example, five LEDs are disposed in a row. For example, by differently configuring light emitting colors of the light emitting elements in the light emitting portion 14 or by combining states such as lighting, extinction or blink, the measuring apparatus 1 may notify the wearer of information relating to an operating mode of the measuring apparatus 1 or measurement of the biometric information.

The band unit 20 extends along the Y-axis direction. The front surface 21a of a main body portion 21 of the band unit 20 is curved in a substantially arc-shape along the top surface 10a of the case unit 10. As the material of the band unit 20, for example, silicone rubber, natural rubber, isoprene rubber, butadiene rubber, styrene butadiene rubber, chloroprene rubber, nitrile rubber, polyisobutylene, ethylene-propylene rubber, chlorosulfonated polyethylene rubber, acrylic rubber, fluororubber, epichlorohydrin rubber, urethane rubber, styrene-based elastomer, olefin-based elastomer, vinyl chloride series elastomer, polyester-based elastomer, polyurethane-based elastomer, silicon-based elastomer, amide-based elastomer, nylon-based elastomer, dynamically crosslinked elastomer or the like, or a material obtained by blending these materials may be used. It is preferable that the band unit 20 have an elastic force capable of applying an appropriate tightening force with respect to the wrist WR and excellent durability and be good for the skin (cause less skin irritation). As a material having such a characteristic, it is preferable that silicone rubber be used.

The band unit 20 includes a main body portion 21 that covers the top surface 10a of the case unit 10, the first band portion 22 that extends from the main body portion 21 in one side of the directions along the side portions 11 of the case unit 10, and the second band portion 24 that extends from the main body portion 21 in the other side of the directions along the side portions 11 of the case unit 10 . The main body portion 21, the first band portion 22, and the second band portion 24 are integrally formed. The main body portion 21 disposed in a middle portion of the band unit 20 is curved along the top surface 10a of the case unit 10, and has the front surface 21a formed by a convex curved surface. The opening portion 21b is formed in the main body portion 21.

The first band portion 22 and the second band portion 24 extend to bend from the curved main body portion 21 toward the bottom surface 10b of the case unit 10. The first band portion 22 includes a tip portion 23 at a free end thereof. The tip portion 23 is bent inward from the first band portion 22 in the extension direction. The second band portion 24 includes a tip portion 25 at a free end thereof. The tip portion 25 is also bent inward from the second band portion 24.

In the first band portion 22, plural adjustment hole portions 26 are provided in a row along the extension direction of the first band portion 22. The plural adjustment hole portions 26 are provided passing through the first band portion 22 in the thickness direction, and are arranged at a substantially uniform arrangement pitch. In the second band portion 24, a connecting portion 27 is provided on the side of the tip portion 25. The connecting portion 27 protrudes inward from the second band portion 24 in a convex shape, and has a connecting hole formed in the width direction of the second band portion 24.

The buckle unit 30 includes a first plate (first buckle portion) 31, a second plate (second buckle portion) 32, and a hinge portion 33 that supports the first plate 31 and the second plate 32 to be rotatable. The buckle unit 30 is a fold-type length adjusting member that connects the first band portion 22 to the second band portion 24. The first plate 31 is connected to the first band portion 22, and the second plate 32 is connected to the second band portion 24. As a material of the buckle unit 30, in a preferred example, stainless steel may be used as a main component. The material is not limited thereto, and a material that has excellent corrosion resistance, satisfies folding durability, and is light in weight may be used. For example, titan may be used. Further, the buckle unit 30 may be formed of resin instead of metal. Thus, a sense of unity of the band unit 20 and the buckle unit 30 increases, to thereby make the appearance fine.

The buckle unit 30 enters the mounted state by being folded so that the second plate 32 overlaps the outer side of the first plate 31. Further, the buckle unit 30 enters the opened state by being expanded so that the second plate 32 is separated outward from the first plate 31.

The case unit 10 and the band unit 20 are integrally provided as the side of the top surface 10a of the case unit 10 is inserted into the opening portion 21b of the band unit 20. A part of the top surface 10a of the case unit 10, the side portions 11, and the end portions 12 are covered by the band unit 20. Further, a part of the top surface 10a of the case unit 10 is covered by a cover portion 16. The cover portion 16 is a decorative sheet in which a portion that overlaps the light emitting portion 14 is opened. The cover portion 16 is formed of a resin film such as polycarbonate, is colored by a colorful material, and is configured to be printable with characters or the like. The cover portion 16 protects the case unit 10 and enhances the degree of freedom in design.

The measuring apparatus 1 includes a control unit, a power supply unit, a communication unit, a sensor unit, and the like in the case unit 10 that is an apparatus main body. These components provided in the case unit 10 are not shown in the figures. The control unit includes a CPU, a ROM, a RAM, and the like, and controls the operation of the measuring apparatus 1 as these hardware components cooperate with software stored in the ROM or the like. The power supply unit includes a power supply circuit, a battery, and the like. The case unit 10 is provided with a terminal portion for charging the battery.

The communication unit performs wireless communication between the measuring apparatus 1 and an external apparatus such as a smart phone or a personal computer, using a known wireless communication method such as Bluetooth (registered trademark), for example. Thus, it is possible to operate the measuring apparatus 1 from the external apparatus, and to transmit the biometric information measured by the measuring apparatus 1 to the external apparatus for storage and management of the biometric information of the wearer. The measuring apparatus 1 has a function of storing the measured biometric information of the wearer and providing the analysis result of the biometric information or information indicating whether the motion quantity is appropriate, for example, to the wearer based on the information, in cooperation with the external apparatus.

The sensor unit includes a tap operating sensor unit (not shown) that detects a tap operation of the wearer in addition to the above-mentioned photoelectric pulse wave sensor unit 5 that detects the biometric information. The photoelectric pulse wave sensor unit 5 includes a light emitting element such as an LED and a light receiving element such as a photodiode, for example. The photoelectric pulse wave sensor unit 5 irradiates the wrist WR of the wearer with detection light from the light emitting element, and receives light reflected from a blood vessel of the wrist WR using the light receiving element, to thereby detect the pulse wave of the wearer. The detection light and the reflected light are respectively output and incident through the window portion 13 which is the detection unit. The measuring apparatus 1 measures the pulse rate of the wearer based on the pulse wave detected in the photoelectric pulse wave sensor unit 5.

The tap operating sensor unit is configured by an acceleration sensor, for example. The tap operation refers to an operation of tapping the measuring apparatus 1 using the finger or palm. The wearer operates the measuring apparatus 1 by performing the tapping operation of tapping the measuring apparatus 1. The measuring apparatus 1 detects the tapping operation of the wearer based on sensor information of the tapping operation sensor unit. In the measuring apparatus 1, a simple operation is performed by the tapping operation, and various settings, specific operations or the like of the measuring apparatus 1 are performed by the external apparatus through wireless communication. With such a configuration, the measuring apparatus 1 does not have to be provided with buttons or switches for the operation. A signal from the acceleration sensor may also be used for reduction processing of body motion noise overlapped with the pulse wave signal when detecting the biometric information.

The measuring apparatus 1 is an apparatus that is not mounted on the wearer only when doing exercise such as walking or running but is constantly mounted on the wearer to measure the biometric information in daily life of the wearer. Thus, it is necessary that the measuring apparatus 1 be capable of measuring the biometric information in a stable state with a small burden (for example, discomfort due to the weight, size or shape of the apparatus, or a tightening force) to the wearer and regardless of the posture or motion of the wearer, even during a period of continuous use. As the measuring apparatus 1 is not provided with the monitor portion (display), the buttons and the switches, it is possible to achieve reduction of the size and weight and increase of a service life of the battery, compared with a general measuring apparatus. Further, when the pulse rate is measured when the wearer does exercise, the body motion noise due to the exercise is overlapped with the pulse wave detected by the detection unit. In order to extract only the pulse wave component from the pulse wave signal with which the body motion noise is overlapped, an acceleration signal output from the acceleration sensor of the tapping operation sensor unit may be used. Thus, the acceleration sensor for detection of the tapping operation and the acceleration sensor for extraction of the pulse wave component can be commonly used by one acceleration sensor, and thus, it is possible to realize reduction of the cost and size and increase of a service life of the sensor.

### Configuration of case unit and band unit

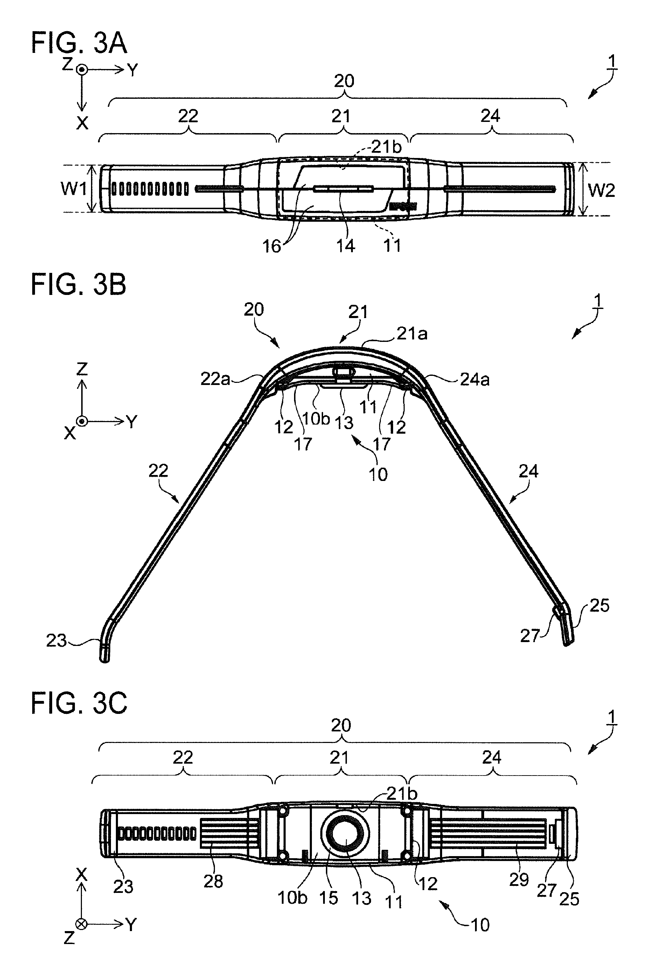

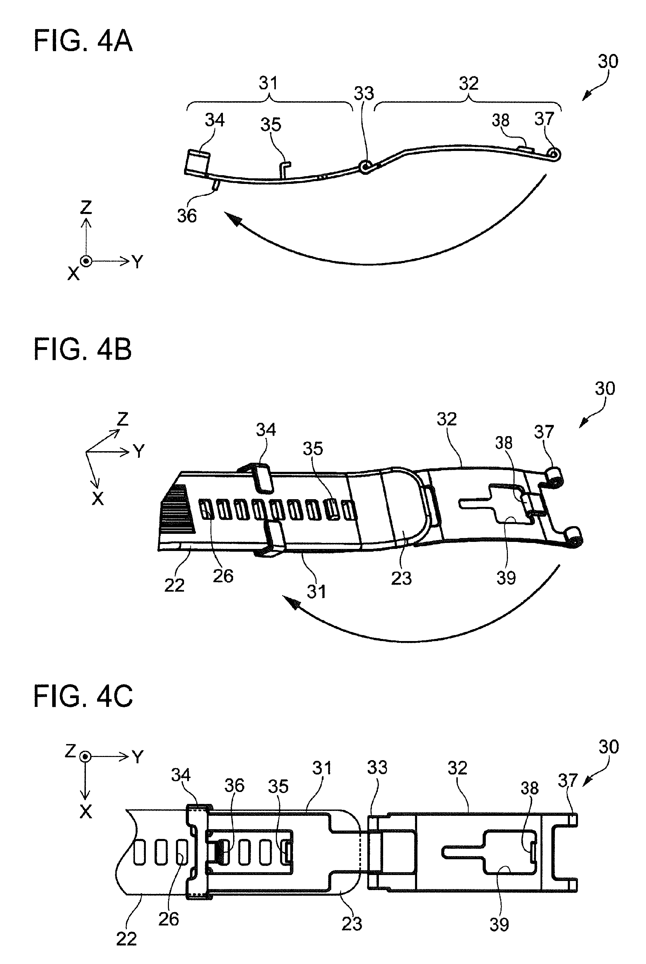

Fig. 3A is a front view of the measuring apparatus, and Fig. 3B is a side view thereof. Fig. 3C is a plan view (rear view) thereof seen from the bottom side (detection unit). Configurations of the case unit 10 and the band unit 20 will be described in detail with reference to Figs. 3A to 3C, and Figs. 4A to 4C.

In the front view shown in Fig. 3A, the top surface 10a (see Fig. 2) of the case unit 10 is covered by the main body portion 21 of the band unit 20 and one pair of cover portions 16. Further, in the front view, the side portions 11 of the case unit 10 are covered by the main body portion 21 of the band unit 20. Accordingly, in the front view, the case unit 10 is not exposed.

Each cover portion 16 is formed in a substantially trapezoidal shape having a top and a bottom along the Y-axis direction. Further, the pair of cover portions 16 is disposed so that the bottoms thereof face each other. Between the pair of cover portions 16, an opening portion that extends along the Y-axis direction is provided, and the light emitting portion 14 is exposed from the opening portion.

The width of the band portion 20 in the extension direction (Y-axis direction) is largest in the main body portion 21. The width of the main body portion 21 is larger than the width of the case unit 10. The width of the first band portion 22 becomes narrow as it is separated from the main body portion 21, and then becomes a predetermined width W1. The width of the second band portion 24 becomes narrow as it is separated from the main body portion 21, and then becomes a predetermined width W2.

In the side view shown in Fig. 3B, in the side portions 11 of the case unit 10, the side of the top surface 10a is covered by the main body portion 21 of the band unit 20, and the side of the bottom surface 10b is exposed. A central portion of the bottom surface 10b is formed by a substantially flat surface, and the end portions 12 positioned at opposite ends of the side portions 11 are projected in the -Z-axis direction (direction where the apparatus is mounted on the wrist WR) from the bottom surface 10b. Accordingly, the bottom surface 10b of the case unit 10 includes a curved surface portion 17 that is bent in a concave shape from the central portion toward the end portions 12. The radius of curvature of the curved surface portion 17 is larger than the radius of curvature of the front surface 21a of the main body portion 21. Since the radius of curvature of the curved surface portion 17 on the inner surface side that contacts with the wrist is larger than the radius of curvature of the front surface 21a on the outer surface side, the thickness of the photoelectric pulse wave sensor unit 5 (see Fig. 2) can be absorbed by expanding the side of the front surface 21a (top surface 10a of the case unit 10), and thus, reduction of the close-contact to the wrist WR of the measuring apparatus 1 is prevented.

Further, the window portion 13 that is the detection unit projected in the -Z-axis direction is provided in the central portion of the bottom surface 10b.

In the plan view shown in Fig. 3C seen from the side of the bottom surface 10b, the case unit 10 of the substantially rectangular shape is fitted into the opening portion 21b (see Fig. 2) provided in the middle portion of the band portion 20. The outer circumference of the case unit 10 is covered by the band unit 20, and the bottom surface 10b is exposed. The window portion 13 provided in the central portion of the bottom surface 10b is formed in a substantially circular shape in the plan view. Further, in the plan view, a bank portion 15 of a ring shape that surrounds the periphery of the window portion 13 is formed outside the window portion 13.

The bank portion 15 is a rib that is integrally formed with the case unit 10, and is provided in a convex shape projected toward the wrist WR from the bottom surface 10b.

The window portion 13 is a transparent convex lens shaped member. In a preferred example, the window portion 13 is formed of transparent resin. Light emitted from the light emitting element of the photoelectric pulse wave sensor unit 5 (see Fig. 2) passes through the window portion 13 and is irradiated onto the wrist WR of the wearer, and light reflected on the blood vessel of the wrist WR passes through the window portion 13 and is received by the light receiving element of the photoelectric pulse wave sensor unit 5. Thus, in order to detect the biometric information in the stable state by the photoelectric pulse wave sensor unit 5, it is preferable that the window portion 13 (detection unit) be stably retained in a state of being close contact with the wrist WR of the wearer in the mounted state.

The first band portion 22 includes plural grooves 28 recessed from the front surface on a side facing the wrist WR (inner side or inner surface). The second band portion 24 includes plural grooves 29 recessed from the front surface on the side facing the wrist WR. The plural grooves 28 and 29 are provided to extend in the Y-axis direction and to be arranged at substantially the same interval in the X-axis direction. As the first band portion 22 and the second band portion 24 include the plural grooves 28 and 29, a substantial area of the first band portion 22 and the second band portion 24 that contacts with the wrist WR of the wearer in the mounted state can be reduced.

Thus, for example, it is possible to improve airing to the wrist WR and to release sweat from the wrist WR, to thereby improve fit of the wearer. Further, by providing the plural grooves 29 in the Y-axis direction, it is possible to prevent deviation of the measuring apparatus 1 in the mounted state in the width direction (X-axis direction crossing the Y-axis direction). In the present embodiment, the opening portion where a part of the side portions 11 of the case unit 10 and the bottom surface 10b are exposed is provided in the main body portion 21 of the band unit 20, but the opening portion where only the window portion 13 is exposed may be provided in the main body portion 21. With such a configuration, since a sense of unity of the case unit 10 and the band unit 20 increases, an excellent design can be achieved.

### Configuration of buckle unit

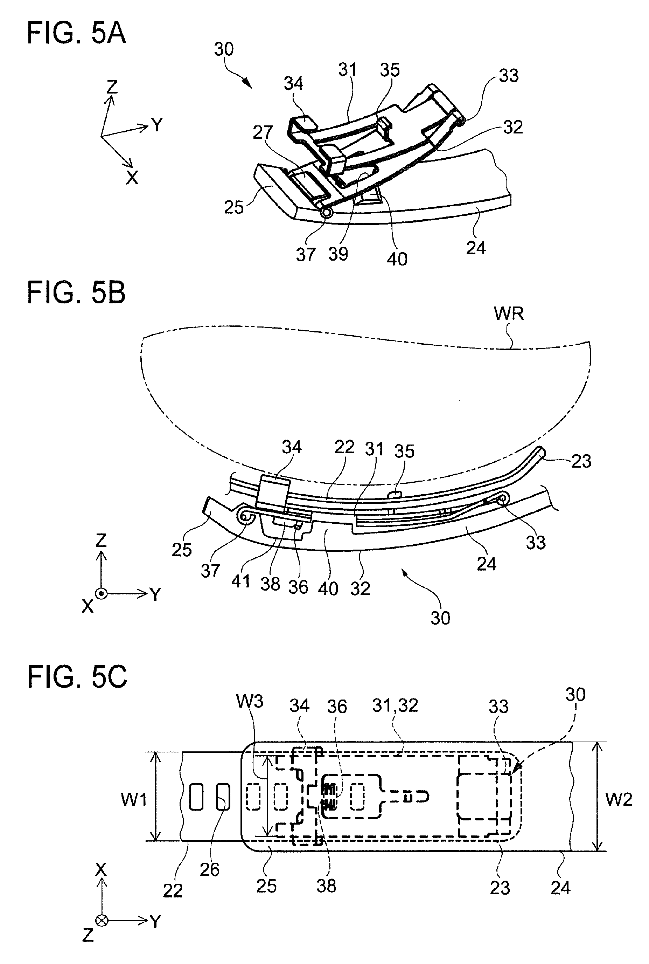

Figs. 4A to 4C and Figs. 5A to 5C are diagrams illustrating a configuration of the buckle unit and a connecting structure with the band unit. Specifically, Fig. 4A is a side view of the buckle unit in the opened state. Fig. 4B is a perspective view illustrating the buckle unit in a state where the first band portion is connected to the first plate, seen from the inner side thereof. Fig. 4C is a plan view illustrating the state shown in Fig. 4B, seen from the outer side thereof. Fig. 5A is a perspective view illustrating the buckle unit in a state where the second band portion is connected to the second plate, seen from the inner side thereof. Fig. 5B is a side view of the band unit and the buckle unit in the mounted state. Fig. 5C is a plan view of the state shown in Fig. 5B, seen from the outer side thereof. Fig. 5B is a schematic cross-section of the wrist WR of the wearer using a two-dotted chain line.

Here, the configuration of the buckle unit 30 and the connecting structure with the band unit 20 will be described with reference to Figs. 4A to 4C and Figs. 5A to 5C.

As shown in Fig. 4A, in the buckle unit 30, one end of the first plate 31 and one end of the second plate are supported to be rotatable by the hinge portion 33. The first plate 31 includes a guide portion 34 provided at the other end thereof, a hook portion 35 that extends inward, and a hook portion 36 that extends outward. The second plate 32 includes a connecting portion 37 provided at the other end thereof, and a convex portion 38 that protrudes inward in the opened state.

The buckle unit 30 enters the mounted state by rotating the connecting portion 37 of the second plate 32 in an arrow direction using the hinge portion 33 as a rotating shaft and folding the second plate 32 to overlap the outer side of the first plate 31 as shown in Fig. 4B. In the mounted state, the hook portion 36 of the first plate 31 is engaged with the convex portion 38 of the second plate 32, and thus, the mounted state where the buckle unit 30 is folded is retained. Further, a positioning hole 39 is formed in the vicinity of the convex portion 38. The positioning hole 39 is a hole of a substantially rectangular shape along the extension direction of the second plate 32.

In the mounted state shown in Fig. 5B, the first band portion 22 is disposed being closest to the side of the wrist WR (inner side), and the first plate 31, the second plate 32, and the second band portion 24 are sequentially disposed toward the opposite side to the wrist WR (outer side). By disengaging the hook portion 36 of the first plate 31 and the convex portion 38 of the second plate 32 from the mounted state and separating the connecting portion 37 of the second plate 32 from the first plate 31 to be expanded to the outer side, the opened state shown in Fig. 4A is obtained.

As shown in Figs. 4B and 4C, the first band portion 22 is connected to the first plate 31 as any one of the plural adjustment hole portions 26 is engaged with the hook portion 35 of the first plate 31. By appropriately selecting the adjustment hole portion 26 to be engaged with the hook portion 35, it is possible to adjust the substantial length of the band unit 20 in the mounted state, and thus, to adjust the tightening force due to the band unit 20. In addition, since the adjustment hole portion 26 is also engaged with the hook portion 35 in the opened state so that the connection state of the first band portion 22 and the first plate 31 is maintained, if the tightening force due to the band unit 20 is once adjusted, the adjusted tightening force is reproduced even though the mounting and detaching to the wrist WR is repeated.

As shown in Fig. 4B, the tip portion 23 of the first band portion 22 is bent inward, that is, toward a side opposite to the first plate 31. Thus, when the first plate 31 and the second plate 32 are folded to enter the mounted state from the opened state, it is possible to prevent the tip portion 23 from bending toward the first plate 31 (outer side) to be wound into between the first band portion 22 and the first plate 31 or into between the second band portion 24 and the second plate 32 (Fig. 5B).

As shown in Fig. 5A, the second band portion 24 is connected to the second plate 32 as the connecting portion 37 and the connecting portion 27 of the second plate 32 are supported to be rotatable by a pin or the like. The tip portion 25 of the second band portion 24 is bent inward, that is, toward the wrist WR. Further, in the second band portion 24, a convex portion 40 is formed at a position that overlaps the positioning hole 39 of the second plate 32 when folded. If the buckle unit 30 is folded and the second plate 32 overlaps the second band portion 24, the convex portion 40 is fitted into the positioning hole 39. Thus, it is possible to prevent position shift of the second plate 32 and the second band portion 24 in the width direction of the second band portion 24. As described above, in the mounted state shown in Fig. 5B, the second band portion 24 is disposed on the outermost side. Since the tip portion 25 of the second band portion 24 is bent inward, it is possible to prevent the tip portion 25 from being caught in a sleeve or the like of clothes in the mounted state.

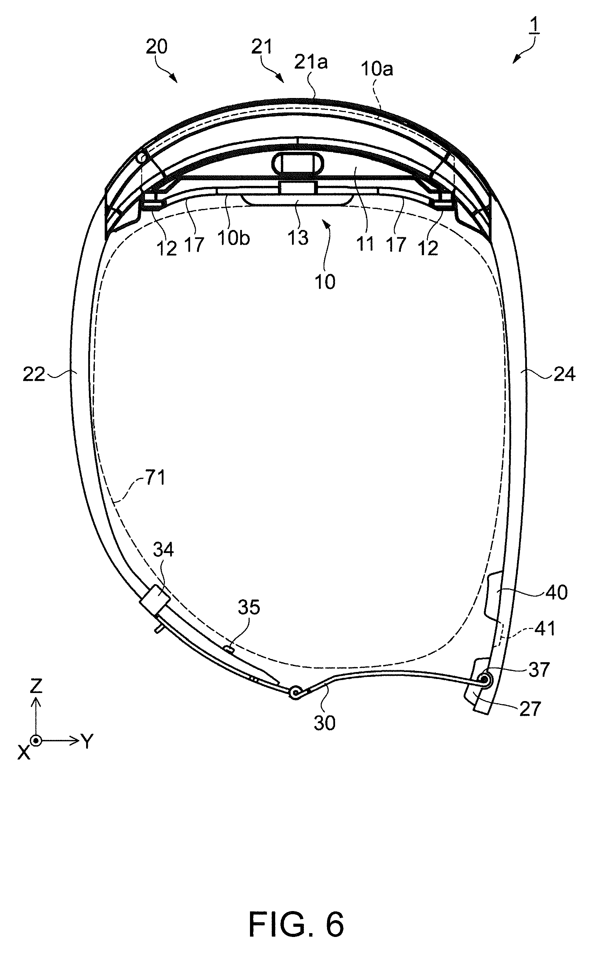

As shown in Fig. 5B, in the second band portion 24, a concave portion 41 is formed at a position that overlaps the hook portion 36 when the apparatus is mounted. The concave portion 41 is formed in a groove shape (see Fig. 6) in a middle portion of the second band portion 24 in the width direction, and releases projection due to the hook portion 36 and the convex portion 38. Thus, floating of the second band portion is prevented.

As shown in Fig. 5C, the width W1 of the first band portion 22 and the width W2 of the second band portion 24 satisfy the relationship of W1<W2. That is, the width W2 of the second band portion 24 disposed outside is wider (larger) than the width W1 of the first band portion 22 disposed inside in the mounted state. Further, when the width of the portion of the buckle unit 30 having the largest width (in the present embodiment, guide portion 34) is represented as W3, it is preferable that W3 be smaller than W2. That is, it is preferable that the width W2 of the second band portion 24 be wider (larger) than the width W3 of the buckle unit 30 disposed on the inner side of the second band portion 24 in the mounted state.

If the width W2 of the second band portion 24 disposed on the outermost side is wider than the width W1 of the first band portion 22 and the width W3 of the buckle unit 30, the first band portion 22 and the buckle unit 30 are covered by the second band portion 24. Thus, in the mounted state, it is possible to prevent the buckle unit 30 formed of a metallic material from being caught in a sleeve or the like of clothes or from contacting with an obstacle or the like, to thereby make the appearance fine.

Here, in at least a portion where the first band portion 22 and the second band portion 24 come into contact with each other, it is preferable that surface texturing, that is, unevenness machining be performed. If the surface texturing is performed in the contacting portion, a frictional force generated when the first band portion 22 and the second band portion 24 rub against each other is reduced to be easily slipped. Thus, compared with a case where the surface texturing is not performed, it is possible to easily perform the mounting and detaching of the measuring apparatus 1 while suppressing winding of the tip portion 23 of the first band portion 22 or the tip portion 25 of the second band portion 24 when the apparatus is mounted.

Further, it is also preferable to perform the surface texturing in portions of the first band portion 22 and the second band portion 24 being in contact with the wrist WR. If the surface texturing is performed in the portions being in contact with the wrist WR, compared with a case where the surface texturing is not performed, it is possible to reduce a substantial contact area per unit area of the first band portion 22 and the second band portion 24. Thus, it is possible to suppress discomfort of the wearer as the first band portion 22 and the second band portion 24 are in close contact with the wrist WR in the mounted state.

### Circular opening size during mounting

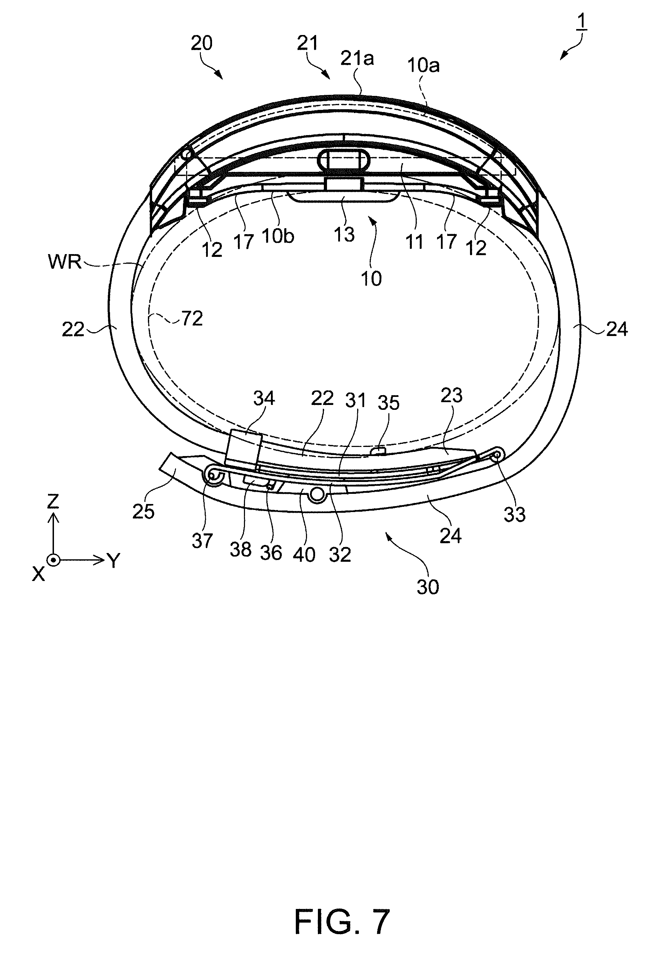

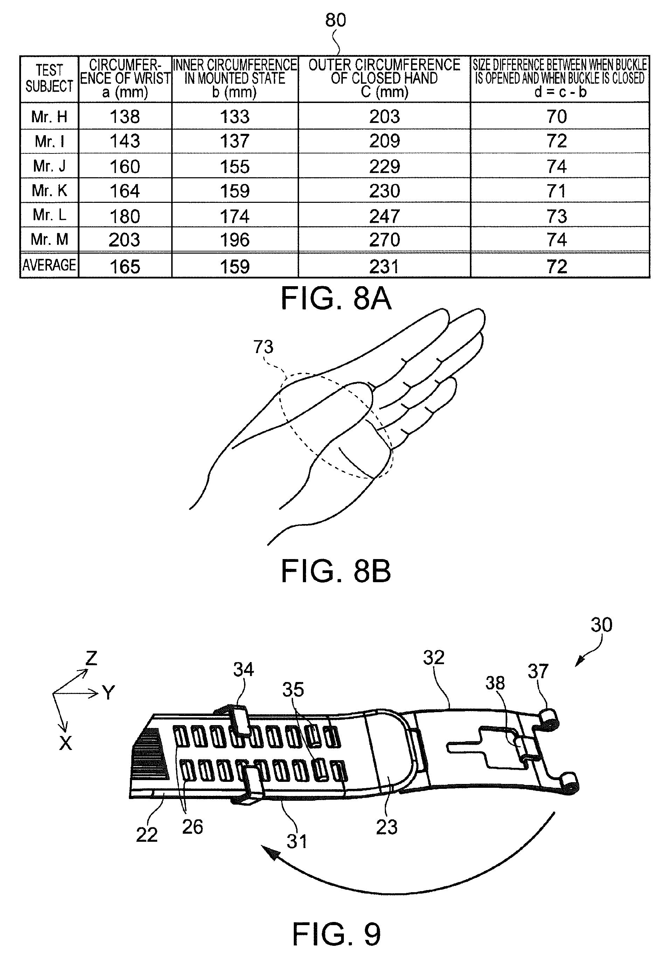

Fig. 6 is a side view of the measuring apparatus in the opened state. Fig. 7 is a side view of the measuring apparatus in the mounted state. Fig. 8A is a table of measured data obtained from plural test subjects. Fig. 8B is a diagram illustrating a state where the hand is closed.

Here, the size of a circular opening portion of the measuring apparatus 1 in the opened state and the mounted state will be described. As described above, in order to stably detect the biometric information, it is necessary that the detection unit be accurately fixed to the detection position of the arm even though the mounting and detaching of the measuring apparatus 1 is repeated. The inventors found an optimal size (length) of the circular opening portion by repeating experiments and verifications. Hereinafter, the description will be made.

Fig. 6 is a side view when the buckle unit 30 of the measuring apparatus 1 is in the opened state, in which an inner circumference 71 is indicated by a dashed line (broken line). Fig. 7 is a side view when the buckle unit 30 is folded to enter the mounted state, in which an inner circumference 72 is indicated by a dashed line.

It is necessary that the measuring apparatus 1 has smooth fit, a function of stably measuring the biometric information after mounting, and a fit property with no stress even during a period of continuous use. The inventors studied a dimension that satisfied these conditions.

Table 80 in Fig. 8A represents results obtained by inspecting the dimension of a mounting portion (wrist), an optimal mounting dimension, or the like for plural test subjects including common adults. Specifically, a circumferential size "a" of the wrist (arm), a length "b" of the inner circumference 72 (Fig. 7) in an optimal mounted state, a length "c" of an outer circumference 73 (Fig. 8B) of the thickest portion of the hand with the hand being closed were measured for each test subject. Data corresponding to representative 6 test subjects is extracted and written in the table 80, but actually, the table is based on data (original data) obtained from more test subjects.

First, the circumferential length "a" of the wrist is in a range of 138 mm to 203 mm and has an average value of 165 mm.

The length "b" of the inner circumference 72 in the optimal mounted state is in a range of 133 mm to 196 mm and has an average value of 159 mm. The inner circumference 72 can be replaced as the circumferential length of the wrist of the wearer in the optimal mounted state.

Here, the reason why the average value 159 mm of the length "b" of the inner circumference 72 in the mounted state is smaller than the average value 165 mm of the circumferential length "a" of the wrist is because tightening necessary for closely fitting the detection unit to the arm with an appropriate strength is performed. That is, it can be understood that it is necessary to shorten the length "b" of the inner circumference 72 by about 5 mm, compared with the circumferential length "a" of the wrist in order to secure close contact of the detection unit. In other words, the dimension of the circular inner circumference formed by the case unit 10, the band unit 20, and the buckle unit 30 is made shorter than the outer circumference of the mounting portion (wrist) of the living body. This dimension corresponds to a setting reverse to that of the above-described watch (a>b), which is a condition necessary for only the measuring apparatus 1.

Next, the length "c" of the outer circumference 73 in a state where the hand is closed is in a range of 203 mm to 270 mm and has an average value of 231 mm. The length "c" may be replaced with the length of the inner circumference 71 (see Fig. 6) in the opened state.

Further, a length adjustment range (dimension difference between when opened and when closed) "d" necessary for the buckle unit 30 becomes a dimension obtained by subtracting the length "b" of the inner circumference 72 in the mounted state from the length "c" of the outer circumference 73 in a state where the hand is closed. The dimension difference "d" is in a range of 70 mm to 74 mm and has an average value of 72 mm. The dimension difference "d" may be replaced with the difference between the ring-shaped inner circumferential dimensions in the opened state (Fig. 6) and the mounted state (Fig. 7).

The inventors regulate design values of the measuring apparatus 1 on the basis of the above result and the above-mentioned original data as follows. In other words, the length of the band unit 20, the number of the adjustment holes, the length of the buckle unit 30, and the like are designed (to be adjustable) to satisfy the following dimensions.

First, the length "b" of the inner circumference 72 in the mounted state shown in Fig. 7 is set in a range of 130 mm to 220 mm. In other words, the length "b" is set to a value in the range of 130 mm to 220 mm. The reason why the lower limit is 130 mm is because if the lower limit is lower than 130 mm, it is too small (too tight) for adults. Further, the reason why the upper limit is 220 mm is because there are rarely persons having a wrist larger than the upper limit and it is possible to cover most common adults with the upper limit. Actual length adjustment is performed by an attachment position of the buckle unit 30 with respect to the first band portion 22 as an initial setting when the measuring apparatus 1 is used. Specifically, in the mounted state, the buckle unit 30 is attached by selecting the position of the adjustment hole so that the length of the inner circumference 72 becomes shorter than the length of the circumference of the wrist WR of the wearer by about 5 mm.

Then, the length "c" of the inner circumference 71 in the opened state shown in Fig. 6 is set in a range of 200 mm to 300 mm. In other words, the length "c" is set to a value in the range of 200 mm to 300 mm. The reason why the lower limit is 200 mm is because if the size is smaller than 200 mm, it is not easy to smoothly mount and detach the apparatus. Further, if the length exceeds 300 mm, it is too large for the common adults. Further, if the size is enlarged, the material of the band unit 20 or the like increases, which causes increase in cost.

Further, the length adjustment range (dimension difference between when opened and when closed) "d" of the buckle unit 30 is set in a range of 70 mm to 80 mm. In other words, the length "d" is set to a value in the range of 70 mm to 80 mm. The reason why the upper limit is 80 mm is because a little margin enables the wearer to easily wear and take off the apparatus. In order to improve the fit in a wearer who has an average size, the length "d" may be set in a range of 72 mm to 78 mm, and may be set to, for example, 74 mm as a value optimal for an individual.

The above-described setting values are examples of setting values for the common adults, and the invention is not limited to the setting values. The values may be differently set according to a mounting target, for example, a gender, an age, and a race. Further, the mounting portion may be a living body portion such as an upper arm, an ankle, the neck or the like.

With the above-described configuration, according to the measuring apparatus 1 of the present embodiment, the following effects can be obtained.

When wearing the measuring apparatus 1, by extending the buckle unit 30, inserting the hand into the ring-shaped large opening portion formed in this state with the hand being closed, and then folding and shortening the buckle unit 30 at the mounting position of the wrist, it is possible to easily wear the measuring apparatus 1. Here, the size (Fig. 7) of the inner circumference 72 in the mounted state is in advance adjusted to a dimension suitable for the wearer as an initial setting according to the attachment position of the buckle unit 30 to the band unit 20. In a preferred example, the window portion 13 (detection unit) comes into close contact with the arm with an interference of about 5 mm.

Accordingly, according to the measuring apparatus 1, it is possible to secure close-contact (fit) necessary for stably measuring the biometric information.

Accordingly, it is possible to provide the measuring apparatus 1 capable of stably measuring the biometric information even though the mounting and detaching is repeated. Further, the close-contact can be secured by the simple method for shortening the buckle unit 30 after mounting. In addition, even in a state where the buckle unit 30 is extended, since the entire apparatus maintains the ring shape, there is no concern about missing the apparatus unless the apparatus is separated from the arm, and it is possible to prevent the apparatus from being broken due to drop.

Further, when the apparatus is mounted on the wrist of the common adults, as described above, the optimal values of the length "b" of the inner circumference 72 in the mounted state, the length "c" of the inner circumference 71 in the opened state, and the length adjustment range "d" of the buckle unit 30 are set on the basis of experimental data. Thus, it is possible to provide the measuring apparatus 1 capable of being comfortably used by most common adults.

The invention is not limited to the above-described embodiment, and various modifications, improvements or the like may be applied to the above-described embodiment. Modification examples will be described as follows.

### Modification example 1

Fig. 9 is a perspective view illustrating a band unit and peripheral parts according to Modification example 1, which corresponds to Fig. 4B.

In the above-described embodiment, the plural adjustment hole portions 26 are formed in the first band portion 22 in a row, but the invention is not limited to the configuration, and the plural adjustment hole portions 26 may be formed in plural rows. For example, as shown in Fig. 9, a configuration in which two rows of plural adjustment hole portions 26 are formed along the extension direction of the first band portion 22 may be used. In a preferred example, the hook portion 35 of the first plate 31 is also formed at two locations to match the two rows.

According to this configuration, since the engagement configuration forms two rows, it is possible to strongly connect the first band portion 22 to the first plate 31, compared with a one-row engagement configuration. Further, a pleasant appearance can be achieved in design.

### Second modification example

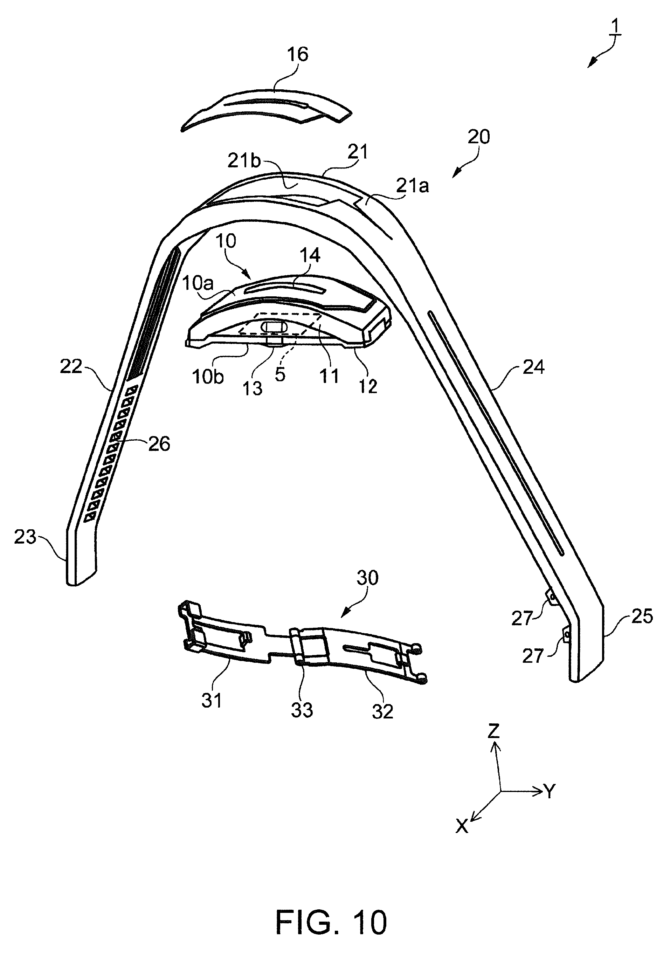

Fig. 10 is an exploded perspective view illustrating a schematic structure of a biometric information measuring apparatus according to Modification example 2, which corresponds to Fig. 2.

In the above-described embodiment, in the second band portion 24, the connecting portion 27 with respect to the buckle unit 30 is formed at one location, but the invention is not limited to this configuration, and the connecting portion 27 may be formed at plural locations. For example, as shown in Fig. 10, the connecting portion 27 may be formed at two locations along the extension direction of the second band portion 24. A difference between Fig. 10 and Fig. 2 is only the number of the connecting portions 27.

According to this configuration, when connecting the second plate 32 to the second band portion 24, any one of the plural connecting portions 27 may be selected for connection. Accordingly, when performing the length adjustment of the ring-shaped opening portion in initial setting, the adjustment cannot be performed only on the side of the first band portion 22, but can also be performed on the side of the second band portion 24. Accordingly, the adjustment width of the ring-shaped opening length becomes large, and thus, the apparatus becomes suitable for use in various people.

### Embodiment 2

Hereinafter, Embodiment 2 of the invention will be described with reference to the drawings.

A biometric information measuring apparatus (hereinafter, referred to as a measuring apparatus) 1B according to Embodiment 2 is an electronic apparatus that is mounted on a living body (for example, a human body) of which biometric information is to be measured and measures the biometric information such as the pulse, similar to the above-described Embodiment 1. Hereinafter, in the following description of Embodiment 2, a configuration different from that of the measuring apparatus 1 of Embodiment 1 will be mainly described. Further, the same reference numerals are given to the same components as those of the measuring apparatus 1 of Embodiment 1, and the description will not be repeated. In the respective drawings shown hereinafter, since components are shown to have sizes capable of being recognized on the drawings, dimensions or ratios of the components may be appropriately different from actual components.

### Overall configuration of biometric information measuring apparatus

The configuration of the measuring apparatus 1B is the same as in Embodiment 1 described with reference to Figs. 1A and 1B, and Fig. 2. Accordingly, the description will not be repeated.

### Configuration of case unit and band unit

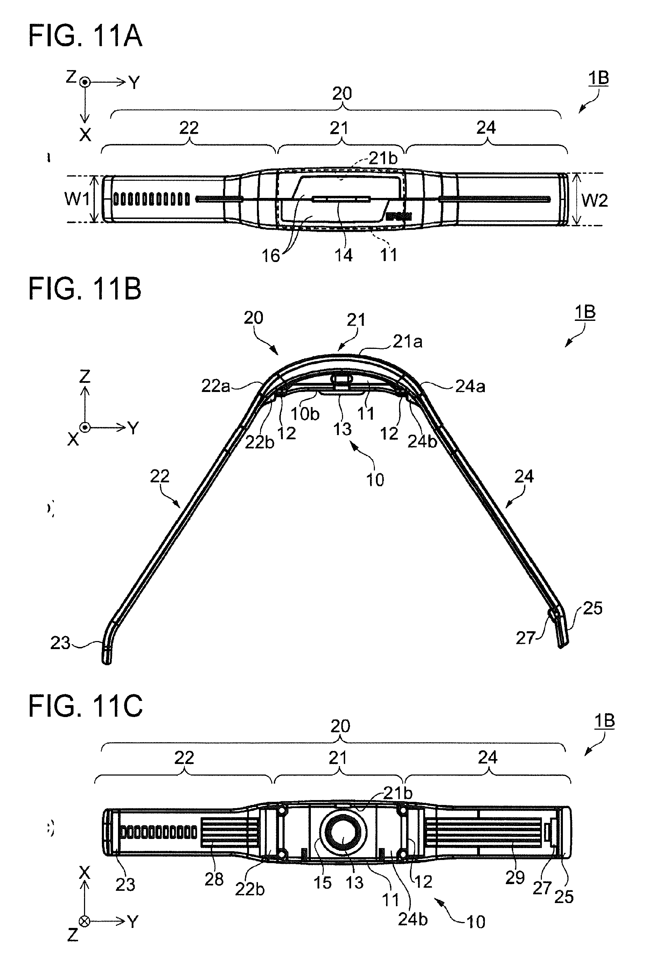

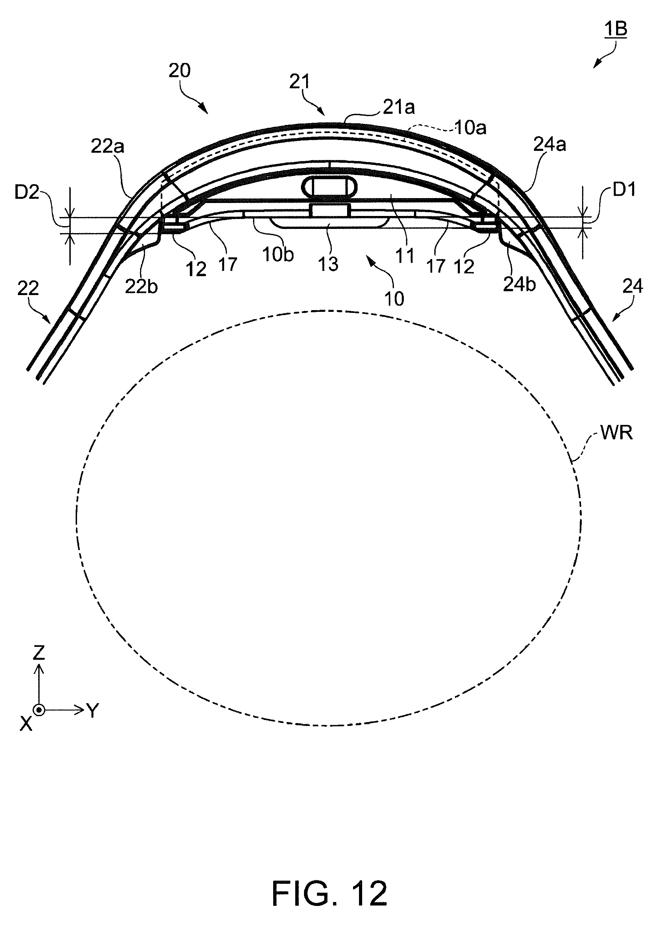

Next, the configuration of the case unit 10 and the band unit 20 of the measuring apparatus 1B according to Embodiment 2 will be described in detail with reference to Figs. 11A to 11C, and Fig. 12. Figs. 11A to 11C and Fig. 12 are diagrams illustrating schematic configurations of the case unit 10 and the band unit 20 of the biometric information measuring apparatus 1B according to Embodiment 2. Fig. 11A is a front view of the measuring apparatus 1B, Fig. 11B is a side view of the measuring apparatus 1B, and Fig. 11C is a plan view of the measuring apparatus 1B seen from the bottom surface 10b mounted on the wrist WR. Fig. 12 is an enlarged side view illustrating the case unit 10 shown in Fig. 11B and the peripheral parts. Fig. 12 shows a cross-section of the wrist WR of the wearer by a two-dotted chain line. Further, the same reference numerals are given to the same components as those of the above-described embodiment, and the description will not be repeated.

As shown in Figs. 11A to 11C, and Fig. 12, the measuring apparatus 1B according to Embodiment 2 includes a convex portion 22b projected in the -Z direction in a portion of the first band portion 22 adjacent to the end portion 12 of the case unit 10. Further, the measuring apparatus 1B includes a convex portion 24b projected in the -Z direction in a portion of the second band portion 24 adjacent to the end portion 12 of the case unit 10.

Specifically, as shown in Fig. 12, the front surface (outer surface) 21a of the main body portion 21 of the band unit 20 is bent in a substantially arc-shape along the upper surface (outer surface) 10a of the case unit 10. The first band portion 22 includes the convex portion 22b projected in the -Z direction in a portion thereof adjacent to the end portion 12 of the case unit 10. The second band portion 24 includes the convex portion 24b projected in the -Z direction in a portion thereof adjacent to the end portion 12 of the case unit 10. Thus, in the mounted state, since the case unit 10 is mounted on the wrist WR in a state of being interposed between the convex portion 22b of the first band portion 22 and the convex portion 24b of the second band portion 24 on opposite sides of the end portions 12, it is possible to stably retain the case unit 10 that is the apparatus main body.

Further, in the mounted state, the inner side of the measuring apparatus 1B is formed in a shape that follows the circumference of the wrist WR by the convex portion 22b of the first band portion 22 and the convex portion 24b of the second band portion 24, and thus, a gap caused between the measuring apparatus 1B and the wrist WR is removed. Thus, for example, when the measuring apparatus 1B is mounted on the wrist WR of the wearer for exercise, it is possible to prevent looseness of the measuring apparatus 1B due to motion of the wearer, and to stably measure the biometric information.

The first band portion 22 includes an outer surface 22a (second curved surface portion) that is bent in a substantially circular shape on the side of the front surface 21a of the portion where the convex portion 22b is provided and is provided to be continuous with the front surface 21a. Further, the second band portion 24 includes an outer surface 24a (second curved surface portion) that is bent in a substantially arc shape on the side of the front surface 21a of the portion where the convex portion 24b is provided and is provided to be continuous with the front surface 21a. The radius of curvature of the outer surface 21a and the radius of curvature of the outer surface 24a are substantially the same, which is smaller than the radius of curvature of the top surface 10a of the case unit 10. Since the main body portion 21 covers the top surface 10a of the case unit 10, the radius of curvature (first radius of curvature) of the front surface 21a (third curved surface portion) of the main body portion 21 is larger than the radius of curvature of the top surface 10a of the case unit 10.

Accordingly, the band unit 20 is bent inside (side where the apparatus is mounted on the wrist WR) with reference to a curved surface obtained by virtually extending the front surface 21a, in the connecting portion of the first band portion 22 and the second band portion 24 to the main body portion 21. Thus, a biasing force that presses the convex portions 22b and 24b toward the end portions from both sides of the case unit 10 and a biasing force that causes the first band portion 22 and the second band portion 24 to be directed inward (direction where the apparatus is mounted on the wrist WR) are applied to the band unit 20.

The bottom surface (bottom surface portion) 10b of the case unit 10 includes the curved surface portion (fourth curved surface portion) 17 between the central portion and the end portions 12 on the opposite sides. The radius of curvature of the curved surface portion 17 is larger than the radius of curvature (first radius of curvature) of the front surface 21a (third curved surface portion) of the main body portion 21. In the measuring apparatus 1B, since the radius of curvature of the curved surface portion 17 on the inner surface side that contacts with the wrist WR is larger than the radius of curvature of the front surface 21a on the outer surface side, the thickness of the photoelectric pulse wave sensor unit 5 (see Fig. 2) that is the detection unit can be absorbed by expanding the side of the front surface 21a (top surface 10a of the case unit 10), and thus, reduction of the close-contact to the wrist WR of the measuring apparatus 1B is prevented.

When the amount of projection of the window portion 13 with respect to the bottom surface 10b is represented as D1 and the amount of projection of the end portions 12 with respect to the bottom surface 10b is represented as D2, in the present embodiment, the relationship of D1<D2 is established, and thus, the end portions 12 are projected toward the wrist WR of the wearer from the window portion 13 with respect to the bottom surface 10b. In other words, the window portion 13 is disposed at a position separated in the +Z direction from the end portions 12 with respect to the wrist WR of the wearer. Accordingly, in the mounted state, the bottom surface 10b of the case unit 10 is easily fitted corresponding to the circle of the wrist WR of the wearer, and thus, it is possible to maintain the window portion 13 in close contact with the wrist WR. Further, the burden of the wearer (for example, feeling of oppression as the window portion 13 is pressed against the wrist WR) is reduced.

It is preferable to set the amount of projection D1 of the window portion 13 and the amount of projection D2 of the end portions 12 with respect to the bottom surface 10b in consideration of the tightening force or the fit to the wrist WR, on the basis of setting of the length of the case unit 10 in the Y-axis direction, the amounts of projection of the convex portions 22b and 24b of the first band portion 22 and the second band portion 24, and the like. The amount of projection D1 of the window portion 13 with respect to the bottom surface 10b of the case unit 10 may be set to be larger than the amount of projection D2 of the end portions 12 (D1>D2).

As described above, according to the configurations of the case unit 10 and the band unit 20 of the measuring apparatus 1B according to the present embodiment, since the bottom surface 10b (window portion 13) of the case unit 10 that is the apparatus main body can be maintained in close contact with the wrist WR in a stable state, even during a period of continuous use of the measuring apparatus 1B, it is possible to stably measure the biometric information.

The case unit 10 is formed in an approximately rectangular shape where the width direction (X-axis direction) is a short side in the front view. Accordingly, the width in a state where the measuring apparatus 1B is mounted on the wrist WR (length in the X-axis direction) can be made to be smaller than that of a general measuring apparatus. Thus, it is possible to reduce the burden of the wearer who wears the measuring apparatus 1B, and to reduce a visual feeling of oppression.

Further, the side of the top surface 10a of the side portion 11 of the case unit 10 and the outer circumference of the case unit 10 are covered by the band unit 20. In other words, the band unit 20 functions as a protection member that covers and protects the circumference of the case unit 10 that is the apparatus main body. Thus, since the case unit 10 does not project toward the front surface 21a from the band unit 20 in the thickness direction and the width direction, it is possible to prevent the case unit from being caught in a sleeve of clothes or contacting with an obstacle or the like.

Further, even when the portion covered by the band unit 20 comes into contact with the obstacle or the like, since the case unit 10 does not directly come into contact therewith, shock to the case unit 10 due to the contact is moderated, and thus, it is possible to prevent the case unit 10 from being separated or deviated from the measurement portion in the wrist WR of the wearer. Thus, even during a period of continuous use, it is possible to suppress the burden of the wearer, and to continuously measure the biometric information in a stable state.

Further, as a part of the case unit 10 is covered by the band unit 20, it is possible to provide a sense of unity in the appearance of the case unit 10 and the band unit 20, and to diversify an external design by combination of the case unit 10, the band unit 20 and the cover unit 16, to thereby make the appearance of the measuring apparatus 1B fine.

### Configuration of band unit and buckle unit

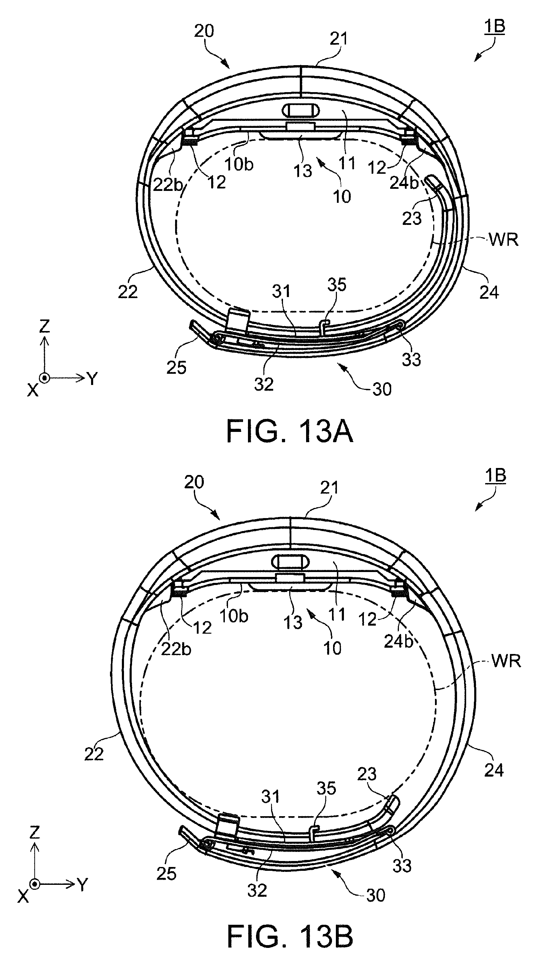

The configuration of the band unit 20 and the buckle unit 30 in the measuring apparatus 1B according to Embodiment 2 is substantially the same as in Embodiment 1 described with reference to Figs. 4A to 4C, and Figs. 5A to 5C. Accordingly, the description about the same configuration will not be repeated, and a different configuration will be described with reference to Figs. 13A and 13B.

Fig. 13A is a side view illustrating a mounted state where the band unit 20 is connected so that the diameter of the band unit 20 that is connected in the ring shape by the buckle unit 30 becomes the smallest. When the first band portion 22 is connected to the first buckle portion (first plate) 31, the adjustment hole portion 26 positioned at the most distant position from the tip portion 23 among the plural adjustment hole portions 26 (see Figs. 5B and 5C), that is, at the closest position to the convex portion 22b is engaged with the hook portion 35 for connection, and thus, the diameter of the connected band portion 20 becomes the smallest. The connecting position is referred to as a first position.

When the first band portion 22 and the first buckle portion 31 are connected to each other at the first position, the length from the position of the adjustment hole portion 26 to be engaged with the hook portion 35 to the convex portion 22b becomes the shortest, and the length from the position of the adjustment hole portion 26 to be engaged with the hook portion 35 to the tip portion 23 becomes the longest. That is, the tip portion 23 of the first band portion 22 disposed inside with reference to the buckle unit 30 and the second band portion 24 becomes the closest to the convex portion 24b of the second band portion 24 and the case unit 10.

Even when the first band portion 22 is connected to the first buckle portion 31 at the first position, the tip portion 23 is disposed to be close to the first buckle portion 31 with reference to the convex portion 24b of the second band portion 24 and the case unit 10. Accordingly, since the tip portion 23 of the first band portion 22 is disposed to be close to the first buckle portion 31 with reference to the case unit 10 where the window portion 13 for detecting the biometric information is provided, the first band portion 22 (tip portion 23) is not interposed between the case unit 10 and the wrist WR, and the first band portion 22 (tip portion 23) does not block the window portion 13.

Further, even when the first band portion 22 is connected to the first buckle portion 31 at the first position, since the tip portion 23 is disposed to be close to the first buckle portion 31 with reference to the convex portion 24b of the second band portion 24, the first band portion 22 is not interposed between the convex portion 24b of the second band portion 24 and the wrist WR. Thus, the measuring apparatus 1B can detect and measure the biometric information in a stable state even in the mounted state where the diameter of the band unit 20 connected in the ring-shape becomes the smallest.

Fig. 13B is a side view illustrating a mounted state where the band unit 20 is connected so that the diameter of the band unit 20 that is connected by the buckle unit 30 in the ring shape becomes the largest. When the first band portion 22 is connected to the first buckle portion 31, the adjustment hole portion 26 positioned at the closest position to the tip portion 23 among the plural adjustment hole portions 26, that is, at the most distant from the convex portion 22b is engaged with the hook portion 35 for connection, and thus, the diameter of the connected band portion 20 becomes the largest. The connecting position is referred to as a second position.

When the first band portion 22 and the first buckle portion 31 are connected to each other at the second position, the length from the position of the adjustment hole portion 26 to be engaged with the hook portion 35 to the convex portion 22b becomes the longest, and the length from the position of the adjustment hole portion 26 to be engaged with the hook portion 35 to the tip portion 23 becomes the shortest. That is, the tip portion 23 of the first band portion 22 disposed inside with reference to the buckle unit 30 and the second band portion 24 becomes the closest to the first buckle portion 31.

Even when the first band portion 22 is connected to the first buckle portion 31 at the second position, the tip portion 23 of the first band portion 22 is disposed to be close to the convex portion 24b of the second band portion 24 and the case unit 10 with reference to the first buckle portion 31. Accordingly, the first band portion 22 is interposed between the first buckle portion 31 and the wrist WR. Thus, since the first buckle portion 31 is not in contact with the wrist WR of the living body even when the diameter of the band unit 20 connected in the ring shape becomes the largest, the burden (discomfort) of the wearer can be suppressed.

### Shape and length of band unit

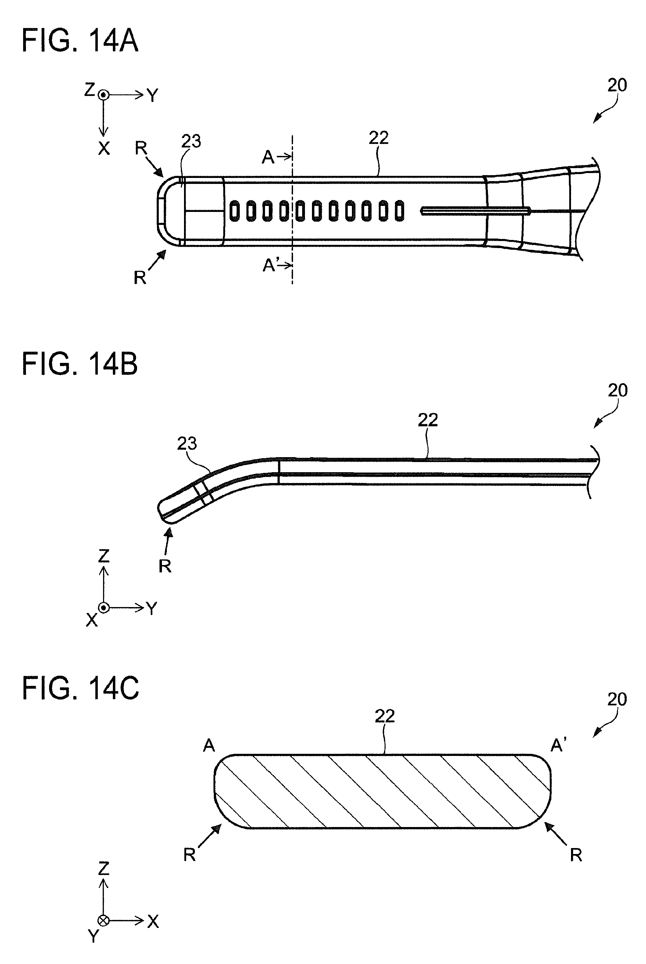

Figs. 14A to 14C are diagrams illustrating shapes of the band unit of the biometric information measuring apparatus according to the present embodiment. Fig. 14A is an enlarged plan view of the first band portion, Fig. 14B is an enlarged side view of the first band portion, and Fig. 14C is a cross-sectional view taken along line A-A' in Fig. 14A.

As shown in Fig. 14A, a corner portion of the tip portion 23 of the first band portion 22 in the plan view is formed to have a curved surface R. Further, as shown in Fig. 14B, a corner portion on the inner surface side of the tip portion 23 of the first band portion 22 in the side view is also formed to have a curved surface R. Further, as shown in Fig. 14C, in the cross-sectional view of the first band portion 22, a corner portion of the first band portion 22 on the inner surface side is also formed to have a curved surface R. This configuration is similarly applied to the second band portion 24.

In this way, since the corner portions of the band unit 20 on the side of the wrist WR of the wearer are formed to have the curved surface, the burden (discomfort) of the wearer during a period of continuous use can be suppressed even though the wrist WR of the wearer is tightened to detect the biometric information.

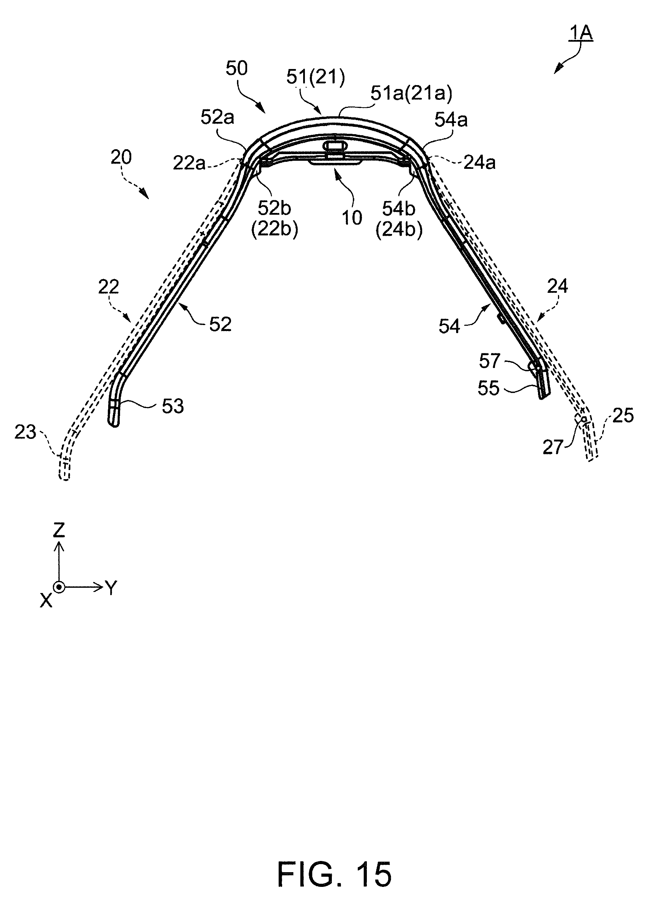

Fig. 15 is a view illustrating comparison of band units having different lengths. Fig. 15 corresponds to the side view shown in Fig. 11B. In a measuring apparatus 1A according to the present embodiment, plural band units having different lengths are prepared. Further, any one is selected from the plural band units, and is combined with the case unit 10 for use. The measuring apparatus 1A shown in Fig. 15 includes a band unit 50, instead of the band unit 20, having a length shorter than that of the band unit 20. In Fig. 15, the band unit 50 is indicated by a solid line, and the band unit 20 is indicated by a broken line.

The band unit 50 is prepared for a wearer having a relative short circumferential length of the wrist WR, for whom the tightening force when the mounting apparatus 1A is mounted using the band unit 20 may be insufficient. The band unit 50 includes a main body portion 51 that covers the top surface 10a of the case unit 10, a first band portion 52 that extends toward one side from the main body portion 51, and a second band portion 54 that extends toward the other side from the main body portion 51. The main body portion 51 includes an outer surface 51a (third curved surface) formed in a convex curved surface. The radius of curvature of the outer surface 51a is substantially the same as the radius of curvature of the front surface 21a of the band unit 20.

Compared with the band unit 20, the length of the first band portion 52 to a tip portion 53 is shorter than the length of the first band portion 22 to the tip portion 23, and the length of the second band portion 54 to a tip portion 55 is shorter than the length of the second band portion 24 to the tip portion 25. The first band portion 52 includes a convex portion 52b projected in the -Z direction in a portion thereof adjacent to the end portions 12 of the case unit 10. The second band portion 54 includes a convex portion 54b projected in the -Z direction in a portion thereof adjacent to the end portion 12 of the case unit 10. Thus, in the mounted state, since the case unit 10 is mounted on the wrist WR in a state of being interposed between the convex portion 52b of the first band portion 52 and the convex portion 54b of the second band portion 54 on opposite sides of the end portions 12, the case unit 10 that is the apparatus main body can be maintained in a stable state. Further, when the wearer wears the measuring apparatus 1A on the wrist WR, since a gap generated between the measuring apparatus 1A and the wrist WR is filled up by the convex portion 52b of the first band portion 52 and the convex portion 54b of the second band portion 54, looseness of the measuring apparatus 1A due to motion of the wearer can be suppressed, and thus, the biometric information can be stably measured.

The first band portion 52 includes an outer surface 52a (second curved surface portion) that is bent in a substantially arc shape on the side of the outer surface 51a of the portion where the convex portion 52b is provided and is provided to be continuous with the outer surface 51a. Further, the second band portion 54 includes an outer surface 54a (second curved surface portion) that is bent in a substantially arc shape on the side of the outer surface 51a of the portion where the convex portion 54b is provided and is provided to be continuous with the outer surface 51a. The radius of curvature of the outer surface 52a and the radius of curvature of the outer surface 54a are substantially the same, which is smaller than the radius of curvature of the outer surface 22a and the outer surface 24a in the band unit 20.

Accordingly, in the band unit 50, compared with the band unit 20, the connecting portions of the first band portion 52 and the second band portion 54, and the main body portion 51 are bent toward the inner surface side from a curved surface obtained by virtually extending the outer surface 51a. Thus, when the circumference of the wrist WR of the wearer is relatively short, the band unit 50 is easily fitted onto the wrist WR, compared with a case where the band unit 20 is used.

In this way, in the present embodiment, any one band unit can be appropriately selected for use from among the plural band units 20 and 50 having different lengths according to the circumference of the wrist WR of the wearer. Thus, an appropriate tightening force can be obtained for wearers having different circumferences of the wrist WR, and thus, it is possible to prevent the case unit 10 from being separated or deviated from the wrist WR of the wearer. Thus, it is possible to suppress the burden (discomfort) of the wearer during a period of continuous use, and to measure the biometric information in a stable state.

Hereinabove, the difference between two band units 20 and 50 having different lengths is described, but a band unit having a different length from the bend units 20 and 50 may be prepared. In this case, in the band unit with a shorter length, the radius of curvature of the second curved surface portion is set to be smaller.

The above-described Embodiment 2 shows only one aspect of the invention, and modifications and applications may be arbitrarily made in the scope of the invention. The following modification examples may be considered, for example.

### Modification example 3

In the measurement apparatus 1B (or 1A) according to Embodiment 2, the case unit 10 and the band unit 20 (or 50) are integrally provided, but the invention is not limited to the embodiment. A configuration in which the case unit 10 is detachably fitted to the band unit 20 (or 50) may be used. With such a configuration in which the case unit 10 is detachably fitted to the band unit 20 (or 50), for example, when the band unit 20 (or 50) is damaged, the band unit 20 (or 50) can be exchanged. Further, when the plural band units having different lengths including the band unit 20 (or 50) are prepared, the band units can be exchanged according to the size of the measurement portion of the wearer. Further, by preparing various band units having different colors, shapes or the like and exchanging the band units according to wearer' s preference, it is possible to diversify an external design.

### Modification example 4

The measuring apparatus 1B (or 1A) according to Embodiment 2 includes the buckle unit 30 that connects the first band portion 22 (or 52) to the second band portion 24 (or 54), but the invention is not limited to this embodiment. For example, a configuration may be used in which a connecting portion for engagement with the adjustment hole portion 26 of the first band portion 22 (or 52) is provided in the tip portion 25 (or 55) of the second band portion 24 (or 54) and the first band portion 22 (or 52) and the second band portion 24 (or 54) are connected to each other by engagement of the connecting portion with the adjustment hole portion 26 of the first band portion 22 (or 52). However, the configuration in which the measuring apparatus 1 includes the buckle unit 30 is preferable in that dropping of the apparatus during mounting or detaching is suppressed and the adjusted tightening force can be maintained even though the mounting and detaching is repeated.

### Modification example 5

In the measuring apparatus 1B according to Embodiment 2, the respective portions of the buckle unit 30 are formed of the metallic materials, but the invention is not limited to this embodiment. For example, the respective portions of the buckle unit 30 may be formed of resin. Thus, a sense of unity of the band unit 20 and the buckle unit 30 is improved, and the appearance of the overall measuring apparatus 1B including the band unit 20 and the buckle unit 30 is further improved.

### Modification example 6

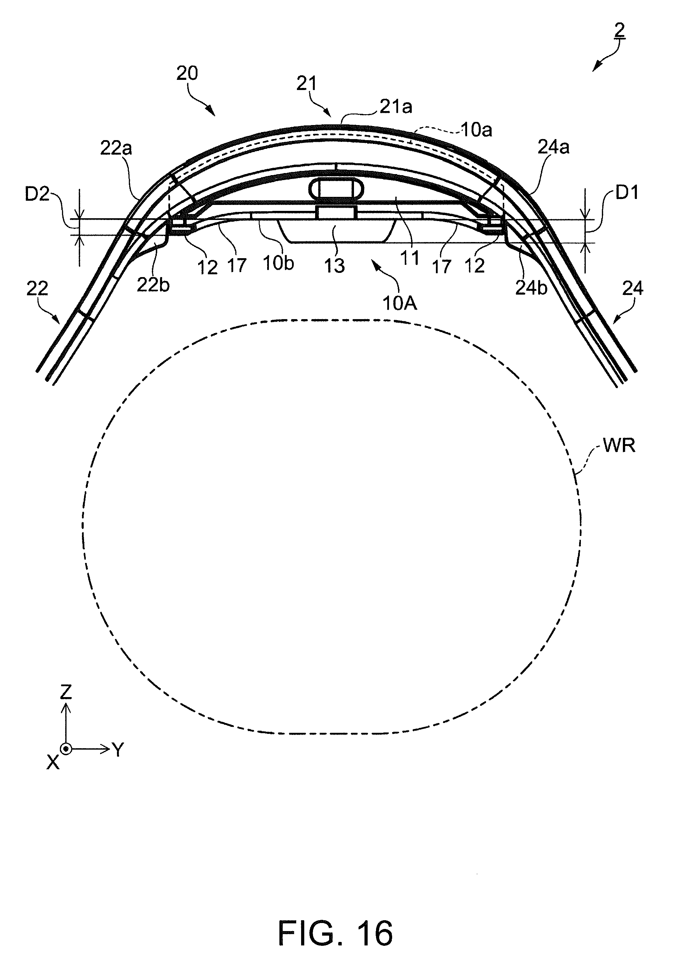

In the measuring apparatus 1B according to Embodiment 2, the window portion 13 is disposed at the position separated toward the +Z direction with reference to the end portion 12, with respect to the wrist WR of the wearer, but the invention is not limited to this embodiment. A configuration in which the window portion 13 is disposed at a position close to the wrist WR of the wearer in the -Z direction with reference to the end portions 12 may be used. Fig. 16 is a diagram illustrating a schematic configuration of a case unit and a band unit of a biometric information measuring apparatus 2 according to Modification example 6. Fig. 16 corresponds to an enlarged side view of the case unit 10 and the peripheral parts shown in Fig. 11B.

As shown in Fig. 16, in the measurement apparatus 2 according to Modification example 6, the amount of projection D1 of the window portion 13 with respect to the bottom surface 10b of a case unit 10A is larger than the amount of projection D2 of the end portions 12 (D1>D2). In other words, in the mounted state, since the window portion 13 is disposed at the position close to the wrist WR of the wearer with reference to the end portions 12, it is possible to strongly press the window portion 13 against the wrist WR of the wearer to come into close contact therewith, compared with the above-described embodiment.

It is preferable that the amount of projection D1 of the window portion 13 with respect to the bottom surface 10b and the amount of projection D2 of the end portions 12 be set in consideration of the tightening force or fit to the wrist WR, on the basis of setting of the length of the case unit 10 in the Y-axis direction, the amounts of projection of the convex portion 22b of the first band portion 22 and the convex portion 24b of the second band portion 24, or the like.

### Modification example 7

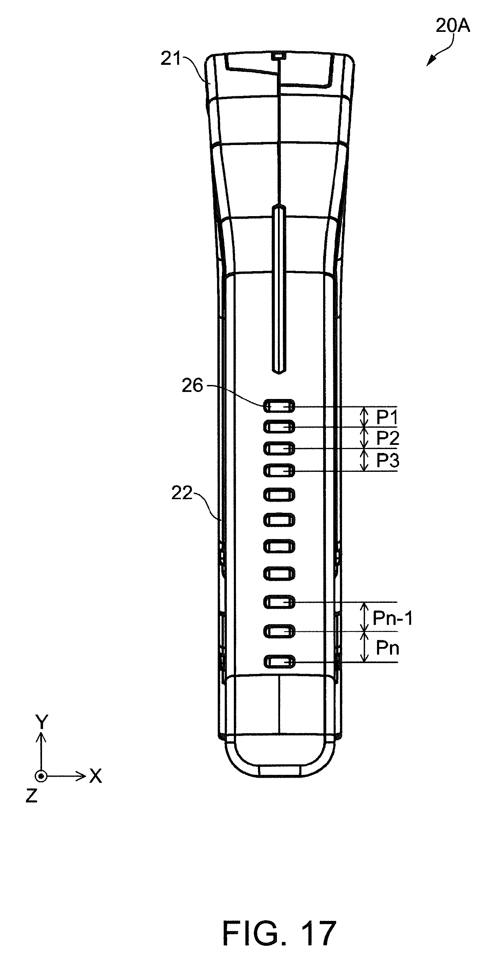

In the measuring apparatus 1B according to Embodiment 2, the arrangement pitch of the plural adjustment hole portions 26 provided in the first band portion 22 is approximately uniform, but the invention is not limited to this embodiment. A configuration in which the arrangement pitch of the plural adjustment hole portions 26 is not uniform may be used. Fig. 17 is a diagram illustrating a schematic configuration of a band unit of a biometric information measuring apparatus according to Modification example 7. Fig. 17 shows a view of the band unit 20A according to Modification example 7 when seen from the outside (side opposite to the wrist WR).

As shown in Fig. 17, in the plural adjustment hole portions 26 of the band unit 20A according to Modification example 7, when a pitch between the adjustment hole portion 26 disposed at a position closest to the main body portion 21 and the next adjustment hole portion 26 is represented as P1, a pitch between the next adjustment hole portion 26 and the second next adjustment hole portion 26 is represented as P2, and a pitch between the second next adjustment hole portion 26 and the third next adjustment hole portion 26 is represented as P3, and similarly, when a pitch between the adjustment hole portion 26 disposed at the most distant position from the main body portion 21 and the next adjustment hole portion 26 is represented as Pn, the relationship of P1<P2<P3<...<Pn-1<Pn is established. That is, in the band unit 20A, the arrangement pitch of the plural adjustment hole portions 26 is set to be smaller as it goes closer to the main body portion 21 (case unit 10).

Since the length from the adjustment hole portion 26 in the first band portion 22 to the main body portion 21 is shortened as the adjustment hole portion 26 for engagement with the hook portion 35 (see Fig. 4B) of the first buckle portion 31 goes closer to the main body portion 21, a tensile stress of the first band portion 22 increases with respect to the same distortion amount (extending length). Thus, if the arrangement pitch of the plural adjustment hole portions 26 is uniform, as the adjustment hole portion 26 for engagement with the hook portion 35 goes closer to the main body portion 21, the increase amount of the tightening force with respect to the wrist WR generated when the adjustment hole portion 26 shifts one by one becomes larger.

As in the band unit 20A according to Modification example 7, with such a configuration in which the arrangement pitch of the plural adjustment hole portions 26 becomes smaller as it goes closer to the main body portion 21, it is possible to suppress the increase amount of the tightening force with respect to the wrist WR generated when the adjustment hole portion 26 shifts one by one from being excessively increased. Thus, it is possible to suppress variation of the tightening forces to the wrists WR due to different sizes of the wrists WR of the wearers or selection of the adjustment hole portions 26. As a result, the case unit 10 (window portion 13) can effectively suppress separation or deviation from the wrist WR of the wearer, and can reduce the burden of the wearer during a period of continuous use.

Further, if the arrangement pitch of the plural adjustment hole portions 26 is uniform, as the adjustment hole portions 26 for engagement with the hook portion 35 goes closer to the main body portion 21, the tensile stress to the first band portion 22 at the position of the adjustment hole portion 26 to be engaged increases. With such a configuration of the band unit 20A of Modification example 7, it is possible to suppress the tensile stress to the first band portion 22 generated when the adjustment hole portion 26 shifts one by one from being excessively increased. Thus, durability of the first band portion 22 (band unit 20) can be enhanced.

A configuration in which the arrangement pitches of all the adjustment hole portions 26 are not set to be non-uniform may be used. For example, a configuration in which the arrangement pitch of the plural adjustment hole portions 26 on a side distant from the main body portion 21 is uniform and the arrangement pitch of the plural adjustment hole portions 26 on a side close to the main body portion 21 is not uniform may be used.

### Modification example 8

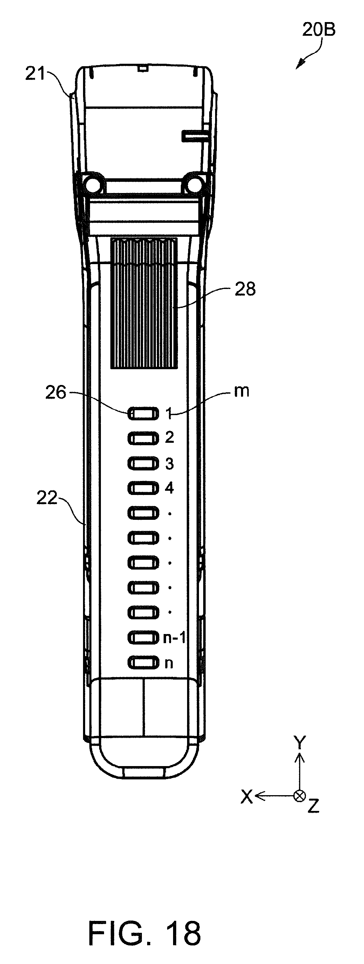

In the measurement apparatus 1B according to Embodiment 2, a configuration in which signs are respectively given to the plural adjustment hole portions 26 may be used. Fig. 18 is a diagram illustrating a schematic configuration of a band unit of a biometric information measuring apparatus according to Modification example 8. Fig. 18 is a diagram of a band unit 20B according to Modification example 8 when seen from the inner side (wrist WR).

As shown in Fig. 18, in the band unit 20B according to Modification example 8, a sign m is given corresponding to each of the plural adjustment hole portions 26 on the inner side of the first band portion 22. The sign m is sequentially given as numbers "1, 2, 3, ..., n-1, n" from the side of the main body portion 21. As the sign m is given to the inner surface of the first band portion 22, when the adjustment hole portion 26 is engaged with the hook portion 35 of the first buckle portion 31 as shown in Fig. 4B, the adjustment hole portion 26 can be individually specified with reference to the sign m. Thus, for example, after the optimal adjustment hole portion 26 is selected to connect the first band portion 22 to the first buckle portion 31, even though the connection is released, it is possible to easily perform re-connection using the previously selected optimal adjustment hole portion 26.

The sign m is not limited to the numbers, and alphabets, codes or the like may be used. Further, the position of the sign m may be a lateral position beside each adjustment hole portion 26, or may be a position between adjacent adjustment hole portions 26. In addition, the sign m may be attached to the outer surface of the first band portion 22.

### Modification example 9

The measurement apparatus 1B according to Embodiment 2 is configured to include the buckle unit 30 that connects the first band portion 22 to the second band portion 24, but the invention is not limited to this embodiment. For example, a configuration in which a connecting portion for engagement with the adjustment hole portion 26 of the first band portion 22 is provided in the tip portion 25 of the second band portion 24 and the first band portion 22 and the second band portion 24 are connected to each other by engagement of the connecting portion with the adjustment hole portion 26 of the first band portion 22 may be used. However, the configuration in which the measuring apparatus 1B includes the buckle unit 30 is preferable in that dropping of the apparatus during mounting or detaching is suppressed and the adjusted tightening force can be maintained even though the mounting and detaching is repeated.

### Modification example 10

In the measuring apparatus 1B according to Embodiment 2, the respective portions of the buckle unit 30 are formed of metallic materials, but the invention is not limited to this embodiment. For example, the respective portions of the buckle unit 30 may be formed of resin. Thus, a sense of unity of the band unit 20 and the buckle unit 30 is improved, the appearance of the overall measuring apparatus 1B including the band unit 20 and the buckle unit 30 is further improved.

### Embodiment 3

Next, Embodiment 3 of the invention will be described with reference to the drawings.

A biometric information measuring apparatus (hereinafter, referred to as a measuring apparatus) according to Embodiment 3 is a heart rate monitoring apparatus that is mounted on a living body (for example, a human body) of which biometric information is to be measured and measures the biometric information such as the pulse (heart rate), similar to the above-described embodiments. Hereinafter, in the following drawings, since respective components are shown to have sizes capable of being recognized on the drawings, dimensions or ratios of the components may be appropriately different from actual components.

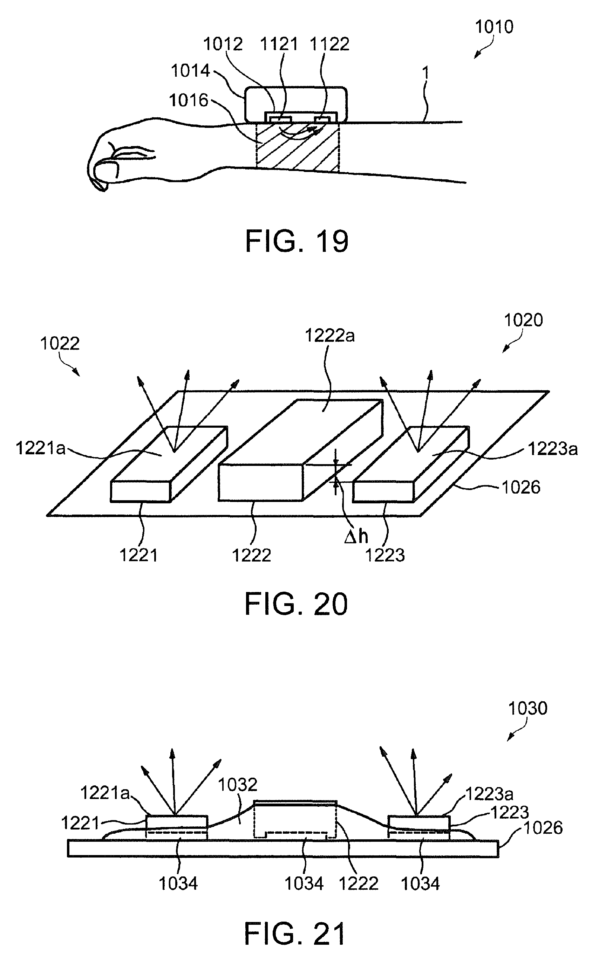

First, before describing the heart rate monitoring apparatus as the biometric information measuring apparatus according to Embodiment 3, a related art example relating to the heart rate monitoring apparatus as the biometric information measuring apparatus according to Embodiment 3 will be described with reference to Fig. 19.

Fig. 19 is a cross-sectional view illustrating a heart rate monitoring apparatus 1010 that is a biometric information measuring apparatus of the related art example that measures a physiologic parameter of a user 1000 who carries a heart rate monitoring apparatus (the arm of the user is shown in Fig. 19). The heart rate monitoring apparatus 1010 includes a sensor 1012 that measures a heart rate that is at least one physiologic parameter of the user 1000, and a case 1014 that accommodates the sensor 1012. The heart rate monitoring apparatus 1010 is mounted on an arm 1001 of the user 1000 by a fixing unit 1016 (for example, hand).

The sensor 1012 includes a light emitting element 1121 and a light receiving element 1122 that correspond to two sensor elements, which serves as a heart rate monitoring sensor for measuring or monitoring the heart rate. However, any sensor that measures one or more physiologic parameters (for example, heart rate, blood pressure, inspired air, skin conductivity, skin moisture or the like) may be used. Further, when the case 1014 includes a band-type housing, for example, the apparatus may be used as a watch-type monitoring apparatus used in sports. Since it is sufficient if the shape of the case 1014 can retain the sensor 1012 at a desired position with respect to the user 1000, the case 1014 may arbitrarily accommodate additional elements such as a battery, a processing unit, a display or a user interface.

The biometric information measuring apparatus in the related art is the heart rate monitoring apparatus 1010 for monitoring the heart rate of the user. Further, the sensor 1012 is an optical sensor that includes the light emitting element 1121 and the light receiving element 1122. The principle of the optical heart rate monitor depends on the light emitting element 1121 (in which an LED is normally used) that is a light source that irradiates light onto the skin. The light irradiated onto the skin is partially absorbed by blood flowing in the blood vessel under the skin, but the remaining light is reflected from the skin to the outside. Further, the reflected light is captured by the light receiving element 1122 (in which a photodiode is normally used). A reception light signal from the light receiving element 1122 represents a signal including information corresponding to the volume of the blood flowing in the blood vessel. The volume of the blood flowing in the blood vessel is changed by the pulse of the heart. In this way, the signal on the light receiving element 1122 is changed corresponding to the heart beat. That is, the change in the signal of the light receiving element 1122 corresponds to the pulse of the heart rate. In addition, by counting the number of pulses per unit time (for example, 10 seconds), the number of heart beats for one minute (that is, heart rate) is obtained.

Hereinafter, a heart rate monitoring apparatus 1020 that is the biometric information measuring apparatus according to Embodiment 3 will be described with reference to Fig. 20. Fig. 20 is a perspective view illustrating the heart rate monitoring apparatus that is the biometric information measuring apparatus according to Embodiment 3.

The heart rate monitoring apparatus 1020 that is the biometric information measuring apparatus according to Embodiment 3 includes a sensor 1022 that includes at least two sensor elements (in this example, three sensor elements including two light emitting elements 1221 and 1223 that are first and second light emitting portions and a light receiving element 1222 that is a light receiving portion). The sensor element detects a sensor signal. The sensor 1022 includes an optical sensor that includes the light emitting elements 1221 and 1223 using two LEDs for light emission to the skin of the user, and at least one light receiving element 1222 (photodiode) for receiving the light reflected from the skin. Further, the heart rate monitoring apparatus 1020 includes a case or a housing (not shown). The case or housing may be similar to or the same as the case 1014 shown in Fig. 19, or may be similar to or the same as the case unit 10 in the above-described Embodiments 1 and 2.

Further, the sensor 1022 is supported on one surface of a carrier (substrate) 1026. Light emitted from the light emitting elements 1221 and 1223 is not absorbed but reflected from the skin or the like, and can directly reach the light receiving element 1222. In the heart rate monitoring apparatus 1020, a distance between the carrier 1026 and top surfaces 1221a and 1223a of the light emitting elements 1221 and 1223 is shorter than a distance between the carrier 1026 and a top surface 1222a of the light receiving element 1222. That is, a difference between the distance between the carrier 1026 and the top surfaces 1221a and 1223a of the light emitting elements 1221 and 1223 and the distance between the carrier 1026 and the top surface 1222a of the light receiving element 1222 is Δh. Further, the light receiving element 1222 receives light through the top surface 1222a that is a top layer. According to this configuration, most of the light emitted from the light emitting elements 1221 and 1223 is directed toward the skin, and the reflected light is directly incident to the light receiving element 1222 without intervention of an air layer or the like. In other words, since the light receiving element 1222 is in close contact with the skin, it is possible to achieve a structure in which a gap is not easily generated between the top surface (light receiving surface) 1222a of the light receiving element 1222 and the skin, to thus prevent light that is a noise source such as external light from being incident to the top surface 1222a. Further, light from the light emitting elements 1221 and 1223 that does not pass through the skin, for example, light that is directly incident to the light receiving element 1222 from the light emitting elements 1221 and 1223 cannot reach the top surface 1222a of the light receiving element 1222.

### Embodiment 4

Next, a biometric information measuring apparatus 1030 according to Embodiment 4 will be described with reference to Fig. 21. Fig. 21 is a front view illustrating the biometric information measuring apparatus 1030 according to Embodiment 4. As shown in Fig. 21, electric connecting terminals 1034 of the light emitting elements 1221 and 1223 and the light receiving element 1222 are preferably covered by an insulating material (for example, epoxy resin) 1032 for protection of electric components. Further, the insulating material 1032 may be provided so as not to cover the light emitting elements 1221 and 1223 or the light receiving element 1222. Specifically, a region between the light emitting element 1221 and the light receiving element 1222, and a region between the light emitting element 1223 and the light receiving element 1222 may be provided to be buried by the insulating material 1223. In other words, the top surface 1222a of the light receiving element 1222 and the top surfaces 1221a and 1223a of the light emitting elements 1221 and 1223 may be provided so as not to be covered by the insulating material 1032. With such a configuration, it is possible to suppress interference due to an air gap between the skin and the light emitting elements 1221 and 1223. Further, the insulating material 1032 may be provided to cover the top surfaces 1221a and 1223a of the light emitting elements or the top surface 1222a of the light receiving element. With such a configuration, it is possible to protect the top surface 1222a of the light receiving element to be in contact with the skin or the top surfaces 1221a and 1223a of the light emitting elements, and thus, it is possible to prevent damage of the top surface 1222a of the light receiving element or the top surfaces 1221a and 1223a of the light emitting elements. In this case, the insulating material 1032 can be considered as a protective film.

In the biometric information measuring apparatus 1030 according to Embodiment 4, as a generally usable example, the insulating material 1032 using epoxy resin is provided. In Fig. 21, the insulating material 1032 is disposed so as not to cover the top surfaces 1221a and 1223a of the light emitting elements 1221 and 1223, and protects the electric connecting terminals 1034. Light emitted from the light emitting elements 1221 and 1223 is indicated by arrows.

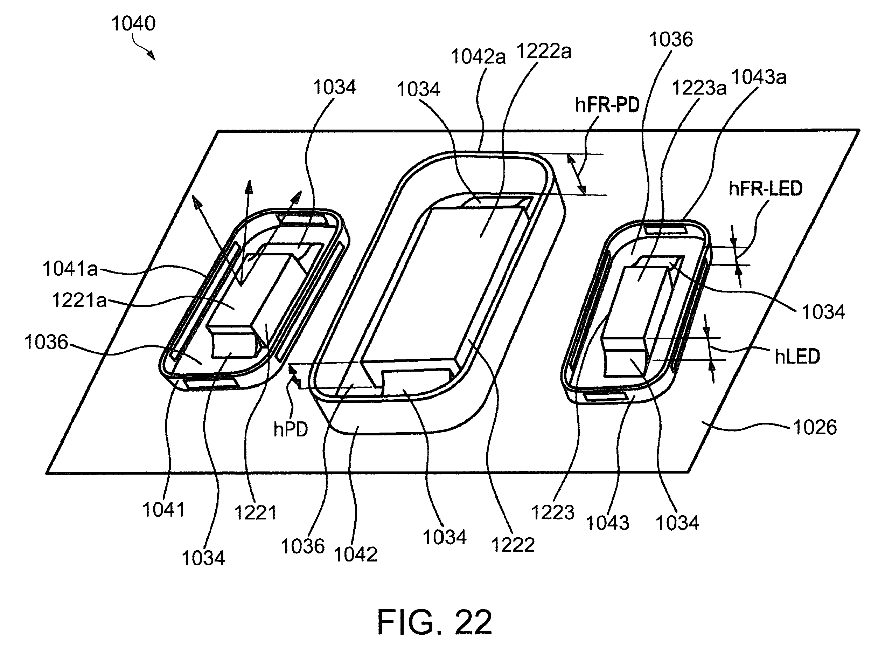

In this way, since the electric connecting terminals 1034 of the light emitting elements 1221 and 1223 and the light receiving element 1222 are protected by performing the arrangement of the insulating material 1032 to the minimum so as not to disturb the correct functions of the biometric information measuring apparatus 1030, the biometric information measuring apparatus 1030 can be further improved. Instead of the configuration of Embodiment 4 in which the epoxy is poured, it is preferable to provide a biometric information measuring apparatus 1040 according to Embodiment 5 as shown in Fig. 22.

### Embodiment 5

Next, the biometric information measuring apparatus 1040 according to Embodiment 5 will be described with reference to Fig. 22. Fig. 22 is a perspective view illustrating the biometric information measuring apparatus according to Embodiment 5. In the biometric information measuring apparatus 1040 according to Embodiment 5, prepared frames 1041, 1042, and 1043 are disposed. The frames 1041, 1042, and 1043 are disposed around the light emitting elements 1221 and 1223 and the light receiving element 1222, and a gap 1036 between the frames 1041, 1042, and 1043, and the light emitting elements 1221 and 1223 and the light receiving element 1222 is formed.

Further, an insulating material (not shown in Fig. 22) is poured using the frames 1041, 1042, and 1043 as a guide, and covers the electric connecting terminals 1034 of the light emitting elements 1221 and 1223 and the light receiving element 1222.

In the example shown in Embodiment 4, the light emitting elements 1221 and 1223 and the light receiving element 1222 are surrounded by the individual frames 1041, 1042, and 1043. As another example, all the frames 1041, 1042, and 1043 may be combined to each other, or all the sensor elements may be surrounded by an integrated frame.

As an improvement point for preventing the influence on the functions of the biometric information measuring apparatus 1040, it is preferable that top edges 1041a and 1043a of the frames 1041 and 1043 around the light emitting elements 1221 and 1223 be lower than the top surfaces 1221a and 1223a of the light emitting elements 1221 and 1223. In other words, a distance hFR-LED between the top edges 1041a and 1043a of the respective frames 1041 and 1043 and the carrier 1026 is the same as or smaller than a distance hLED between the top surfaces 1221a and 1223a of the light emitting elements 1221 and 1223 surrounded by the respective frames 1041 and 1043 and the carrier 1026 (hFR-LED≤hLED).

Preferably, a difference between the distance hFR-LED between the top surfaces 1221a and 1223a of the light emitting elements 1221 and 1223 and the carrier 1026 and the distance hLED between the top edges 1041a and 1043a of the frames 1041 and 1043 and the carrier 1026 is set in a range of 0.1 mm to 0.8 mm. More preferably, the difference between the distance hFR-LED between the top surfaces 1221a and 1223a of the light emitting elements 1221 and 1223 and the carrier 1026 and the distance hLED between the top edges 1041a and 1043a of the frames 1041 and 1043 and the carrier 1026 is set in a range of 0.2 mm to 0.5 mm.

Further, it is preferable that a top edge 1042a of the frame (receiver frame) 1042 around the light receiving element 1222 be higher than the top surface 1222a of the light receiving element 1222. In other words, a distance hFR-PD between the top edge 1042a of the frame 1042 and the carrier 1026 is larger than a distance hPD between the top surface 1222a of the light receiving element 1222 surrounded by the frame 1042 and the carrier 1026 (hFR-PD≤hPD).

Preferably, a difference between the distance hFR-PD between the top surface 1222a of the light receiving element 1222 and the carrier 1026 and the distance hFR-PD between the top edge 1042a of the frame 1042 and the carrier 1026 is set in a range of 0 mm to 0.5 mm. More preferably, the difference between the distance hFR-PD between the top surface 1222a of the light receiving element 1222 and the carrier 1026 and the distance hFR-PD between the top edge 1042a of the frame 1042 and the carrier 1026 is set in a range of 0.1 mm to 0.2 mm.

The distance hFR-PD between the top edge 1042a of the frame 1042 and the carrier 1026 is larger than the distance hFR-LED between the top surfaces 1221a and 1223a of the light emitting elements 1221 and 1223 and the carrier 1026 (hFR-PD>hFR-LED).

For example, when the light receiving element 1222 and the light emitting elements 1221 and 1223 are provided to be close to each other, a configuration in which only a single frame wall is present between the light receiving element 1222 and each of the light emitting elements 1221 and 1223 may be used. This configuration may be usable in view of easiness of manufacturing. When the single frame wall forms a case, frame walls of the frame of the light receiving element 1222 and the frame of each of the light emitting elements 1221 and 1223 are integrally formed. This means that the frame walls of the light emitting elements 1221 and 1223 become higher. Specifically, in each of the frames 1041 and 1043 that surround the light emitting elements 1221 and 1223, the frame wall on a side thereof where the light receiving element 1222 is present is high, and the other frame wall is lower than the top surfaces 1221a and 1223a of the light emitting elements 1221 and 1223.

Further, instead of the frames 1041, 1042, and 1043, a configuration in which a first wall portion is provided between the light receiving element 1222 and the light emitting element 1221 or 1223 and a second wall portion is provided outside the light emitting elements 1221 and 1223, that is, on a side opposite to the first wall portion with respect to the light receiving element 1222 may be used.

In this configuration, a distance between the carrier 1026 and a top surface of the first wall portion may be set to be larger than a distance between the carrier 1026 and a top surface of the second wall portion. With such a configuration, it is possible to realize the function of the frames with less members, compared with a case where the light emitting elements and the light receiving element are surrounded as shown in Fig. 22.

As in Embodiment 5, by using the frames 1041 and 1043 or the frame 1042, it is possible to prevent an insulating material such as poured epoxy resin from flowing out. Further, the division of the insulating material such as epoxy resin using such an additional structure corresponds to an option for enabling high mass productivity. The frames 1041, 1043 and 1042 maybe formed of the same material as that of the carrier 1026. For example, the frames may be formed using epoxy resin or polycarbonate resin by injection molding.

As described above, the insulating material 1032 (see Fig. 20) protects the electric connecting terminals 1034 of the sensor elements (light emitting elements 1221 and 1223, and light receiving element 1222). However, the electric connecting terminals 1034 should be in close contact with additional electronic devices (for example, driver, detection electronics, processor or power source) that are different components. Thus, this means that any electric connection to the additional electronic devices is present in the carrier 1026 (or printed circuit board (PCB)).

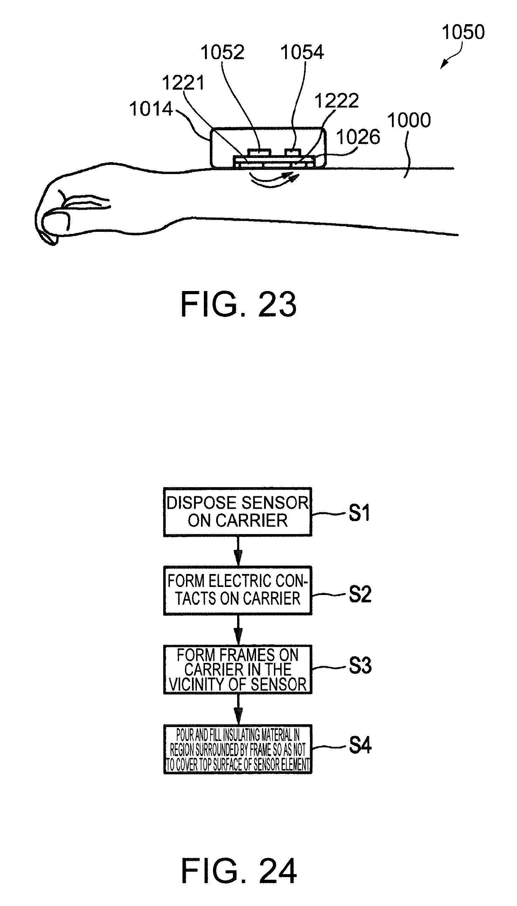

A biometric information measuring apparatus according to Embodiment 6 will be described with reference to Fig. 23. Fig. 23 is a cross-sectional view illustrating the biometric information measuring apparatus according to Embodiment 6. The biometric information measuring apparatus 1050 according to Embodiment 6 includes the above-described additional electronic devices (for example, processor 1052 and driver 1054). An external electric connecting terminal (not shown) is not disposed in the same carrier 1026 as the sensor elements (light emitting element 1221 and light receiving element 1222). That is, the additional electronic devices are disposed on a carrier or substrate different from the sensor elements. With such a configuration, it is possible to mount necessary additional electronic devices on the biometric information measuring apparatus 1050 while maintaining good contact between the skin and the sensor elements (light emitting element 1221 and light receiving element 1222). For example, the external electric connecting terminal may be disposed on a side surface of the carrier 1026.

As described above, various types of sensors may be used in the biometric information measuring apparatus according to the invention. For example, when the light receiving element 1222 is an electric sensor, two skin conductance electrodes (for example, sensor elements (light emitting element 1221 and light receiving element 1222 shown in Fig. 20)) that are in contact with the skin of the user and measure the conductance of the user are covered with the skin. Further, two or more types of sensors may be used in this kind of biometric information measuring apparatus. That is, the number of sensor elements is not limited.

A flowchart illustrating a method for manufacturing the biometric information measuring apparatus that measures physiologic parameters, as described in Embodiment 3 to Embodiment 6, is shown in Fig. 24.

In step S1, the sensor 1022 that includes at least two sensor elements (light emitting element 1221 and light receiving element 1222) for detecting a sensor signal is disposed on the carrier 1026. In step S2, electric contacts of the sensor elements are formed on the carrier 1026. In step S3, one or more frames 1041 and 1042 are formed on the carrier 1026 in the vicinity of the sensor 1022 and/or respective sensor elements (light emitting element 1221 and light receiving element 1222). In step S4, the insulating material 1032 is poured and filled in regions surrounded by the respective frames 1041 and 1042 so as not to cover the top surfaces 1221a and 1222a of the sensor elements (light emitting element 1221 and light receiving element 1222) provided in the carrier 1026.

According to Embodiment 3 to Embodiment 6, the method capable of protecting the electric contacts without badly affecting the performance of the biometric information measuring apparatus is provided. Further, the method capable of protecting the performance of the sensor is provided. At least one of the frames 1041 and 1043 prevents shift of the position of the sensor over the entire skin. Further, at least one of the frames 1041 and 1043 can prevent direct emission light from being input to the light receiving element 1222. Preferably, the heights of the frames 1041 and 1043 around the light emitting elements 1221 and 1223 on the side directing toward the light receiving element 1222 should be smaller than the heights of the top surfaces 1221a and 1223a of the light emitting elements 1221 and 1223. In addition, the frame 1042 around the light receiving element 1222 may be higher than the top surface 1222a of the light receiving element 1222.

## Claims

1. A biometric information measuring apparatus comprising:
a case unit that is provided with a detection unit that detects biometric information;
a band unit that fixes the case unit to a living body; and
a buckle unit that is connected to the band unit.

2. The biometric information measuring apparatus according to claim 1, wherein
a hole portion is provided in the band unit,
the case unit is fitted in the hole portion,
the band unit includes a first band portion that extends from the hole portion toward one end thereof and a second band portion that extends from the hole portion toward the other end thereof, in an extension direction of the band unit, and
the buckle unit is attached to connect the first band portion and the second band portion.

3. The biometric information measuring apparatus according to claim 1 or claim 2, wherein
the band unit is formed of a material including resin having elasticity, and
the buckle unit is formed of metal.

4. The biometric information measuring apparatus according to any of claims 1-3, wherein
the buckle unit is a fold-type buckle that includes a hinge portion, and
the size of an inner circumference of a ring shape formed by the case unit, the band unit and the buckle unit in an extended state, in an opened state where the buckle unit is opened, is in a range of 200 mm to 330 mm.

5. The biometric information measuring apparatus according to claim 4, wherein
the size of the inner circumference of the ring shape formed by the case unit, the band unit and the buckle unit, in a mounted state where the buckle unit is folded, is in a range of 130 mm to 220 mm.

6. The biometric information measuring apparatus according to claim 4 or claim 5, wherein
the size of the inner circumference of the ring shape formed by the case unit, the band unit and the buckle unit in the mounted state is shorter than the size of an outer circumference of a mounting portion of the living body.

7. The biometric information measuring apparatus according to any of claims 4-7, wherein
a difference between the sizes of the inner circumference of the ring shape in the opened state and the mounted state is in a range of 70 mm to 80 mm.

8. The biometric information measuring apparatus according to any of claims 1-7, wherein
the buckle unit includes a first plate and a second plate around the hinge portion,
the second plate is connected to the second band portion, is provided with a positioning hole, and is folded to overlap the second band portion in the mounted state, and
a convex portion is provided in the second band portion at a position for fitting in the positioning hole.

9. The biometric information measuring apparatus according to claim 8, wherein
the first plate is connected to the first band portion, and includes a hook portion for engagement with the second plate in the folded state, and
a concave portion is provided in the second band portion at a position that overlaps the hook portion in the mounted state.

10. The biometric information measuring apparatus according to claim 8 or claim 9, wherein
a plurality of adjustment holes for position adjustment is provided in the first band portion along the extension direction,
a hook portion for engagement with the adjustment holes is provided in the first plate, and
the plurality of adjustment holes is provided to form at least one row along the extension direction, and the number of the hook portion is the same of the number of the row.

11. The biometric information measuring apparatus according to any of claims 8-10, wherein
a first connecting portion for connection to the second band portion is provided in the second plate,
a plurality of second connecting portions is provided in the second band portion along the extension direction, and
the second plate is fixed to the second band portion through any one of the plurality of second connecting portions.

12. The biometric information measuring apparatus according to any of claims 1-11, wherein
the detection unit includes a first light emitting portion, a second light emitting portion, and a light receiving portion.

13. The biometric information measuring apparatus according to claim 12, wherein
the detection unit includes a carrier portion, and the first light emitting portion, the second light emitting portion, and the light receiving portion are disposed on a surface of the carrier portion.

14. The biometric information measuring apparatus according to claim 13, wherein
a distance between the carrier portion and a top surface of the light receiving portion is larger than a distance between the carrier portion and a top surface of the first light emitting portion.
